# EUROPEAN PATENT SPECIFICATION

(11) **EP 0 636 174 B1**
(45) Date of publication and mention of the grant of the patent: **14.01.2004**
(21) Application number: 93907733.5
(22) Date of filing: 13.04.1993
(51) Int. Cl.: C12N 15/12, C07K 14/705, C07K 7/06, C07K 16/28, A61K 38/08, A61K 38/17, A61K 39/395

(54) **HYALURONAN RECEPTOR (RHAMM = RECEPTOR FOR HYLURONAN MEDIATED MOBILITY) AND HYALURONAN BINDING PEPTIDES**
HYALURONAN REZEPTOR (REZEPTOR DURCH HYALURONAN VERURZACHTE BEWELICHKEIT) UND HYALURONAN BINDENDE PEPTIDE
RECEPTEUR D'HYALURONANE (RHAMM = RECEPTEUR POUR LA MOBILITE DUE A L'HYALURONANE) ET PEPTIDES DE LIAISON A L'HYALURONANE

(30) Priority: 09.04.1992 GB 9207949
(43) Date of publication of application: 01.02.1995
(73) Proprietor: UNIVERSITY OF MANITOBA, Winnipeg, Manitoba R3T 2N2 (CA); MANITOBA CANCER TREATMENT AND RESEARCH FOUNDATION, Winnipeg, Manitoba R3E 0V9 (CA)
(72) Inventor: TURLEY, Eva, Ann, Winnipeg, Manitoba R3B 0R4 (CA)
(74) Representative: Bannerman, David Gardner
(86) International application number: PCT/CA1993/000158
(87) International publication number: WO 1993/021312

(56) References cited:
- THE JOURNAL OF CELL BIOLOGY vol. 112, no. 5, March 1991, pages 1041 - 1047 TURLEY, E.A. ET AL.; 'Hyaluronan and a cell-associated hyaluronan binding protein regulate the locomotion of Ras-transformed cells.' cited in the application
- DEVELOPMENTAL BIOLOGY vol. 146, 1991, pages 186 - 197 BARNEJEE, S.D. ET AL.; 'Monoclonal antibody to chick embryo hyaluronan-binding protein : Changes in distribution of binding protein during early brain development.' See Introduction,
- BIOCHEMISTRY. vol. 26, 1987, EASTON, PA US pages 2997 - 3005 TURLEY, E.A. ET AL. 'Characterization of Hyaluronate binding proteins isolated from 3T3 and murine sarcoma virus transformed 3T3 cells' cited in the application
- THE JOURNAL OF CELL BIOLOGY vol. 117, no. 6, June 1992, pages 1343 - 1350 HARDWICK C;HOARE K;OWENS R;HOHN HP;HOOK M;MOORE D;CRIPPS V;AUSTEN L;NANCE DM;TURLEY EA; 'Molecular cloning of a novel hyaluronan receptor that mediates tumor cell motility'
- THE JOURNAL OF CELL BIOLOGY vol. 105, 1987, NEW YORK, USA pages 2403 - 2408 GOETINCK P.F. ET AL. 'The tandemly repeated sequences of cartilage link protein contain the sites for interaction with hyaluronic acid'
- THE JOURNAL OF BIOLOGICAL CHEMISTRY. vol. 262, no. 36, 1987, BALTIMORE US pages 17757 - 17767 DOEGE, K. ET AL.; 'Complete primary structure of the rat cartilage proteoglycan core protein deduced from cDNA clones'
- BIOCHEMICA ET BIOPHYSICA ACTA vol. 1078, no. 1, 30 May 1991, AMSTERDAM, NL pages 12 - 18 FORSBERG N;GUSTAFSON S; 'Characterization and purification of the hyaluronan-receptor on liver endothelial cells.'

## Description

The present invention relates to a novel hyaluronan receptor protein involved in cell motility and to novel hyaluronan binding peptides. It relates also to methods for controlling cell locomotion and for controlling or treating physiological conditions involving cell locomotion.

In the description which follows, references are made to certain literature citations which are listed at the end of the specification.

### Background of the Invention

Increased synthesis of hyaluronan (HA) has been associated with the morphogenesis of many tissues (38), and with wound repair, tumour invasion and immune recognition (27,39,40). Further, HA has been proposed to regulate cell locomotion and cytodifferentiation that occur during these phenomena (33, 43, 44). HA likely mediates these effects either by interacting with specific HA receptors on the cell surface (27,39) or with certain hyaluronan binding proteins in the extracellular milieu. High affinity cell binding sites for HA have previously been demonstrated by kinetic studies on a variety of cell types (27).

A number of cell associated and extracellular HA binding proteins, including link protein (48,47,8), CD44 (89, 23, 24, 73, 71, 76, 79), aggrecan (72), versican (52), GHAP (85), collagen type VI (81) and TSG-6 (51), have been characterized. In link protein, two tandem repeat loops have been found to contain HA binding sites and peptides mimicking specific sequences within these regions have been shown to bind to HA (8).

Prior to the work of the present inventor, however, the amino acid sequence requirements for HA binding had not been recognised.

It has been observed that the transforming oncogene H-ras promotes mammalian cell locomotion (17), although the regulatory mechanisms were unknown. Several observations suggested that these mechanisms are complex and involve, at least, the release of autocrine motility factor(s) (14, 20), growth factors (14) and the glycosaminoglycan, hyaluronan (HA) (20,34). In particular, HA appears to function as an autocrine mechanism for stimulating maximal locomotion in ras transformed cells (34). Further, HA is also required for the ability of an autocrine motility stimulating factor, to promote breast carcinoma cell locomotion (20).

HA promoted, ras transformed cell locomotion requires the presence of a novel hyaluronan receptor complex termed HARC (34). This complex of proteins occurs at the cell surface or is released as soluble proteins of MWE 72, 68, 58 and 52 kDa proteins (29). The complex is tightly regulated in vitro (32) and expressed on the leading lamellae and perinuclear region only on rapidly locomoting cells (28,31). Both polyclonal and monoclonal antibodies prepared against this complex block cell locomotion regulated by mutant ras (34).

The nature of the HA receptor involved in cell locomotion had not been described prior to the work of the present inventor.

### DESCRIPTION OF DRAWINGS

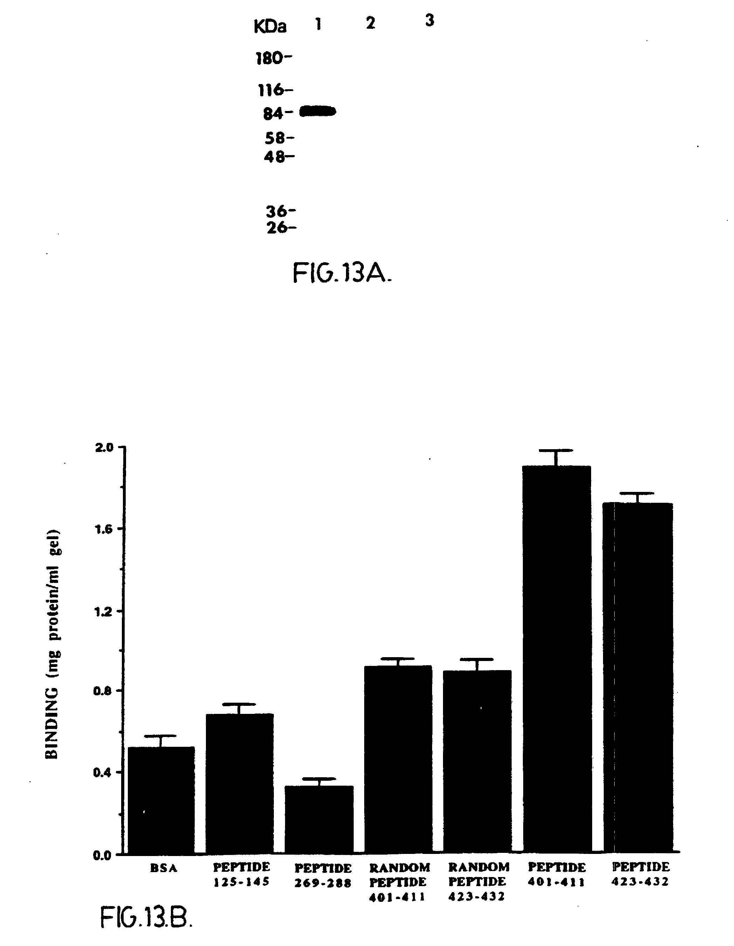

Certain embodiments of the invention are described, reference being made to the accompanying drawings wherein:
Figure 1 shows a restriction map of a 2.9 Kb cDNA clone encoding the HA receptor of the invention.
Figure 2 shows the ultrastructural localisation of the protein encoded by the cDNA of the invention.
Figure 3 shows the immunofluorescent localisation of the encoded protein relative to HARC proteins. A : Mab 3T3-5 which specifically reacts with 52-58 kD proteins of HARC. B : Pab to Peptide II.
Figure 4 shows:
   Panel A: immunoblots of fractionated proteins probed with Pab to Peptide II as primary antibody.
   Panel B: immunoblots of fractionated proteins probed with RAS - 10 antibody as primary antibody. Primary antibodies were detected by chemiluminescence. Protein standards are marked by arrowheads and include phosphorylase b (97.4 kDa), bovine serum albumin (68 kDa) and trypsin inhibitor f (21.5 kDa).
   Panel C: Northern blot of RNA hybridized with a ³²P-labelled 1.4 kb cDNA fragment containing the open reading frame under high stringency.
   Panel D: Blots of panel C re-probed with H-GAPDH to control for RNA loading..

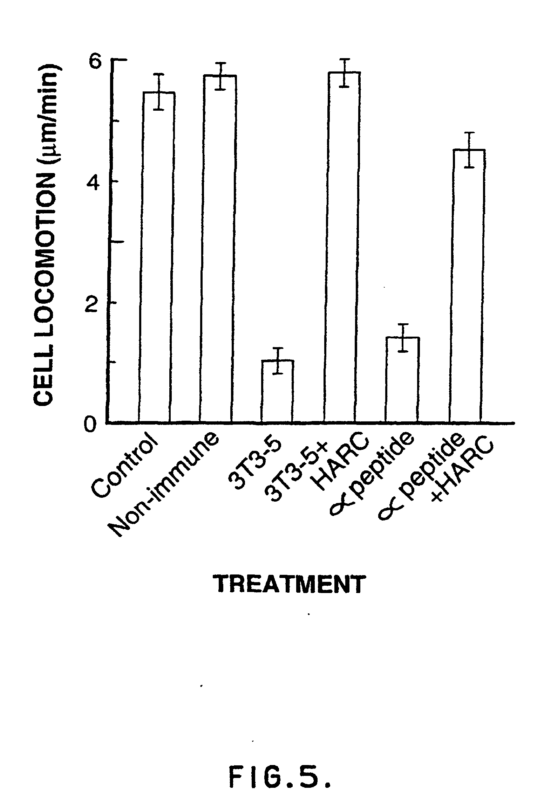

Figure 5 shows effect of antibodies on HA - promoted locomotion in ras-transformed fibroblast cells.

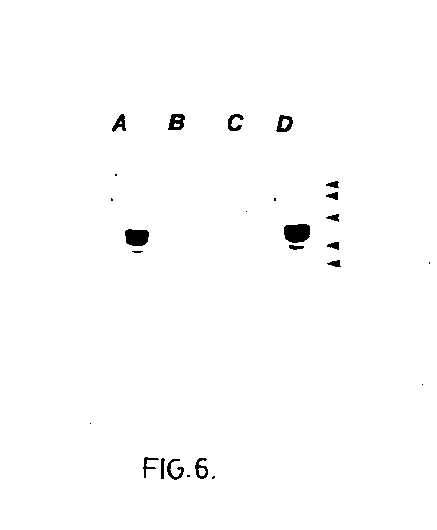

Figure 6 shows the binding of bacterially expressed RHAMM to biotinylated HA and to antibody to HARC proteins.
   - Lane A:: Biotinylated HA added in absence of HA.
   - Lane B:: Biotinylated HA added in presence of 100-fold excess unlabelled HA.
   - Lane C:: control bacterial plasmid with no RHAMM encoding insert.
   - Lane D:: Lysate incubated with Mab 3T3-5 to the 58, 52 kDa HARC proteins.

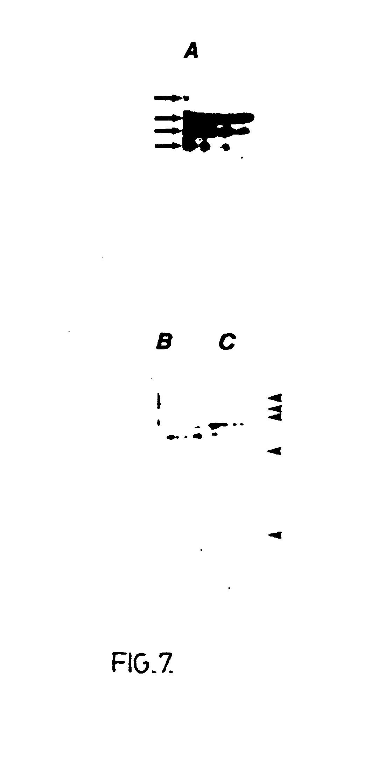

Figure 7 shows:
   - Panel A:: HARC proteins visualised with India ink.
   - Panel B:: Immunoblot of HARC proteins reacted with Mab 3T3-5.
   - Panel C:: Immunoblot of HARC proteins reacted with Pab to Peptide T.

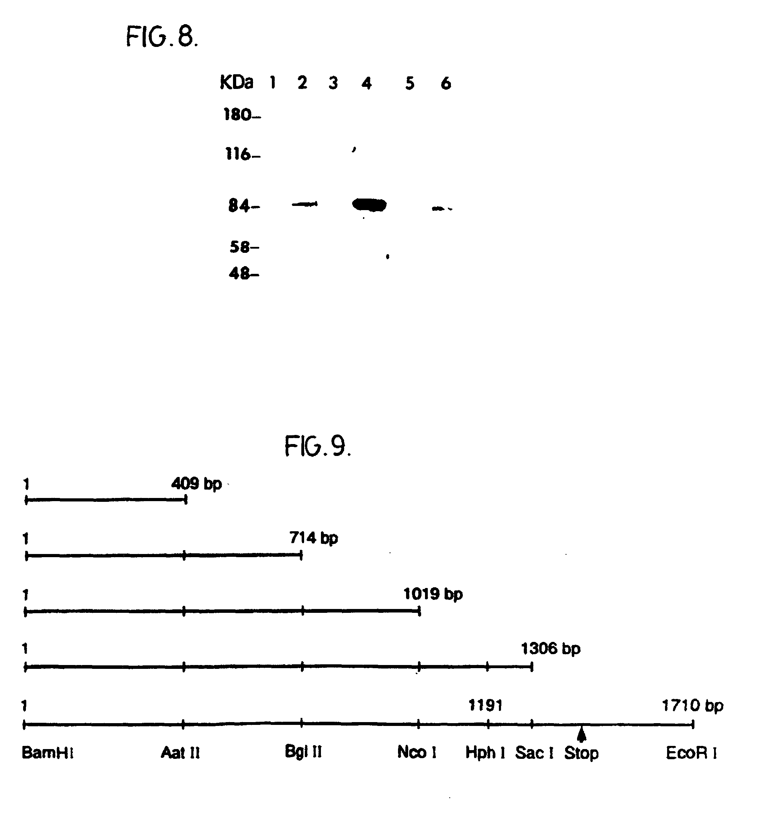

Figure 8 shows Western blot assays of GST-RHAMM fusion proteins expressed in E. coli reacted with:
   - Lane 2:: Pab to a peptide encoded in RHAMM cDNA (peptide ¹²⁵⁻¹⁴⁵)
   - Lane 4:: Mab to RHAMM;
   - Lane 6:: biotin-labelled HA
   Lanes 1, 3 and 5 show preparations from lysates of E. Coli cells containing the pGEX-2T plasmid without the RHAMM cDNA insert, reacted with the reagents of Lanes 2, 4 and 6 respectively.
Figure 9 shows the truncation strategy for preparing fusion products spanning the RHAMM cDNA.

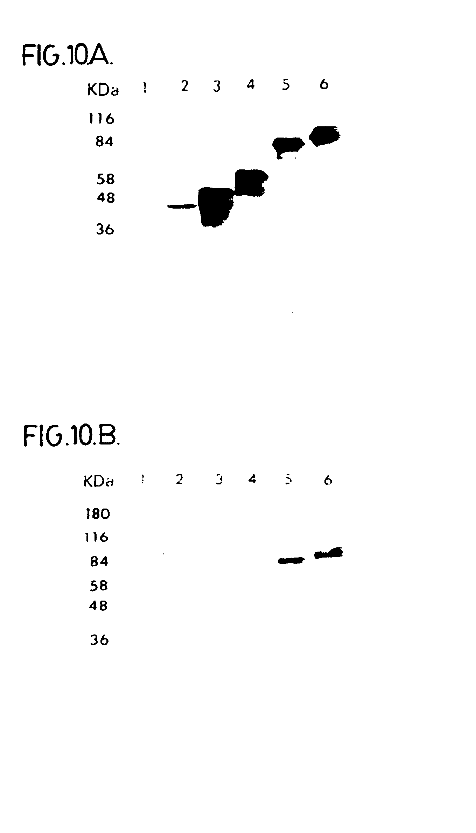

Figure 10A shows visualisation of fusion proteins from truncated RHAMM cDNAs using anti-GST antibodies
   - Lane 1:: HB101 lysate;
   - Lane 2:: fusion protein made from cDNA truncated with AatII + EcoRI;
   - Lane 3:: truncated with BglII + EcoRI;
   - Lane 4:: truncated with Ncol + EcoRI;
   - Lane 5:: truncated with SacI + EcoRI;
   - Lane 6 :: untruncated RHAMM cDNA.
Figure 10B shows fusion proteins as in Figure 10A visualised using biotin-labelled HA.

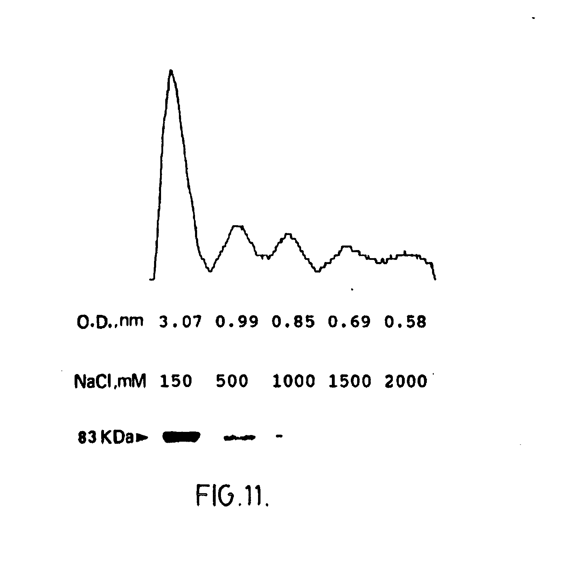

Figure 11 shows effect of NaCl concentration on interaction of HA and RHAMM.

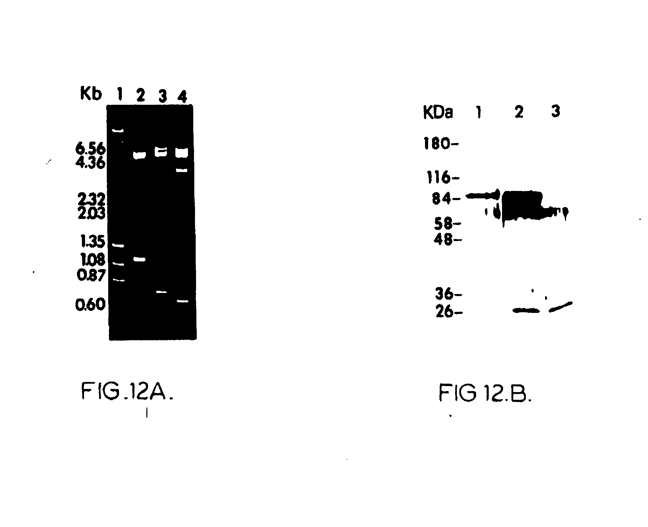

Figure 12A shows agarose gel electrophoresis of truncated RHAMM cDNAs:
   - Lane 1:: DNA markers;
   - Lane 2:: pGEX-2T containing RHAMM cDNA;
   - Lane 3:: as in 2 but truncated with SacI and EcoRI;
   - Lane 4:: as in 2 but truncated with HpbI and EcoRI;
Figure 12B shows transblots of fusion proteins from truncated RHAMM cDNAs binding to biotin-labelled HA:
   - Lane 1:: complete RHAMM fusion protein;
   - Lane 2:: fusion protein from RHAMM cDNA truncated with SacI and EcoRI;
   - Lane 3:: fusion protein from RHAMM cDNA truncated with HphT and EcoRI
Figure 13A shows competition with HA binding to RHAMM fusion protein by:
   - Lane 1:: BSA (3mg/ml)
   - Lane 2:: peptide^{aa 401-411} (3mg/ml)
   - Lane 3:: peptide ^{aa 423-432} (3mg/ml)
Figure 13B shows binding of various peptides to HA-Sepharose affinity gel.

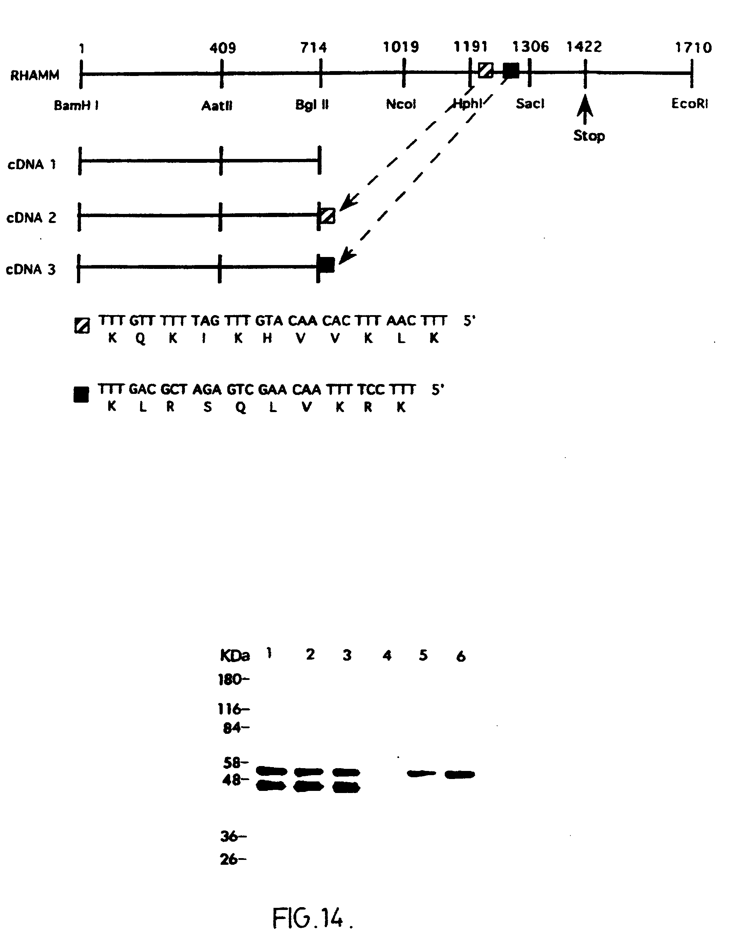

Figure 14A shows construction of recombinant RHAMM containing HA binding domains, peptide^{aa 401-411} (Sequence ID No. 3) ( 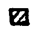 ) and peptide^{aa 423-432} ( Sequence ID No. 4) (■). Corresponding nucleotide sequences: Sequence Nos. 20 and 21.
Figure 14B shows trans blots of GST-RHAMM fusion proteins visualised with Pab to Peptide^{aa 125-145} (Lanes 1 - 3) or biotin-labelled HA (Lanes 4 - 6):
   - Lanes 1,4:: GST fusion non-recombinant polypeptide^{aa 1-238};
   - Lanes 2,5:: peptide^{aa 401-411} linked to polypeptide^{aa 1-238};
   - Lanes 3,6:: peptide^{aa 423-432} linked to polypeptide^{aa 1-238}.

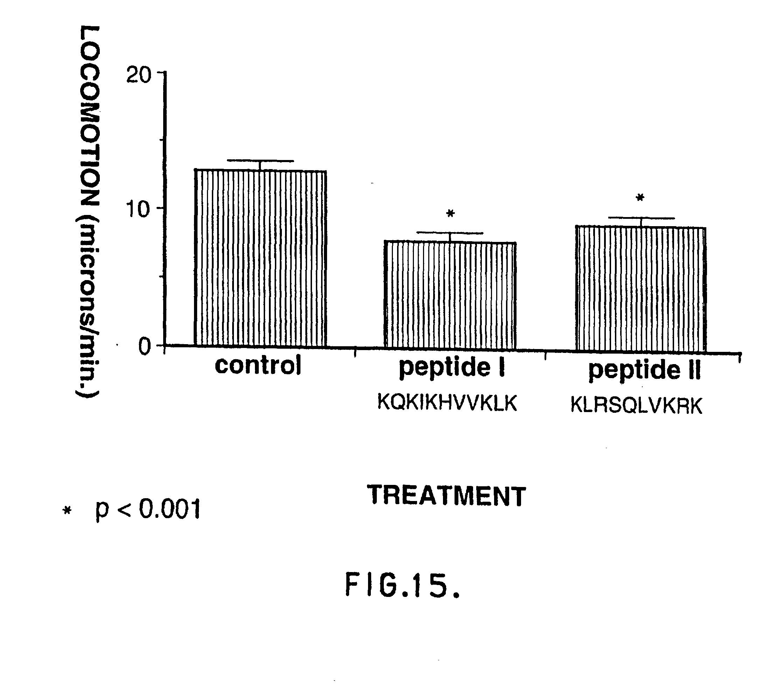

Figure 15 shows effect of RHAMM peptides on locomotion of ras-transformed fibroblast cells.

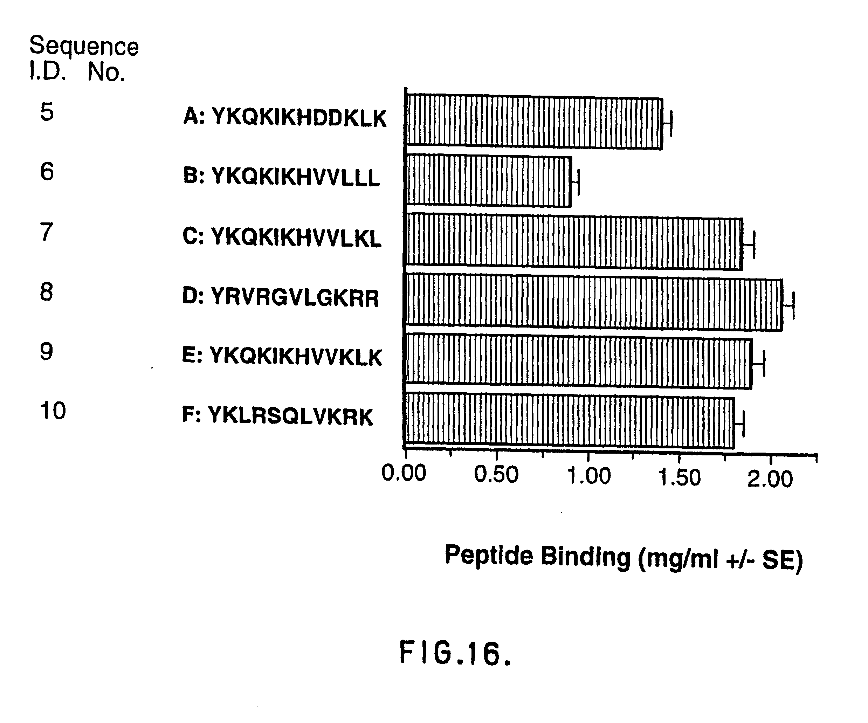

Figure 16 shows affinity chromatography of synthetic peptides on HA-sepharose.

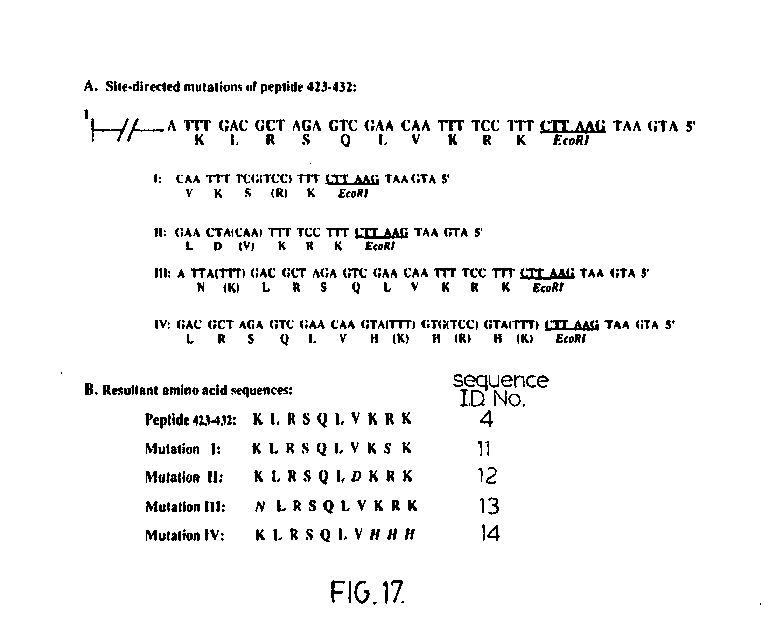

Figure 17A shows the strategy of site-directed mutagenesis of the HA-binding domain, peptide^{aa423-432}, of RHAMM; Figure 17B shows the resultant amino acid sequences.

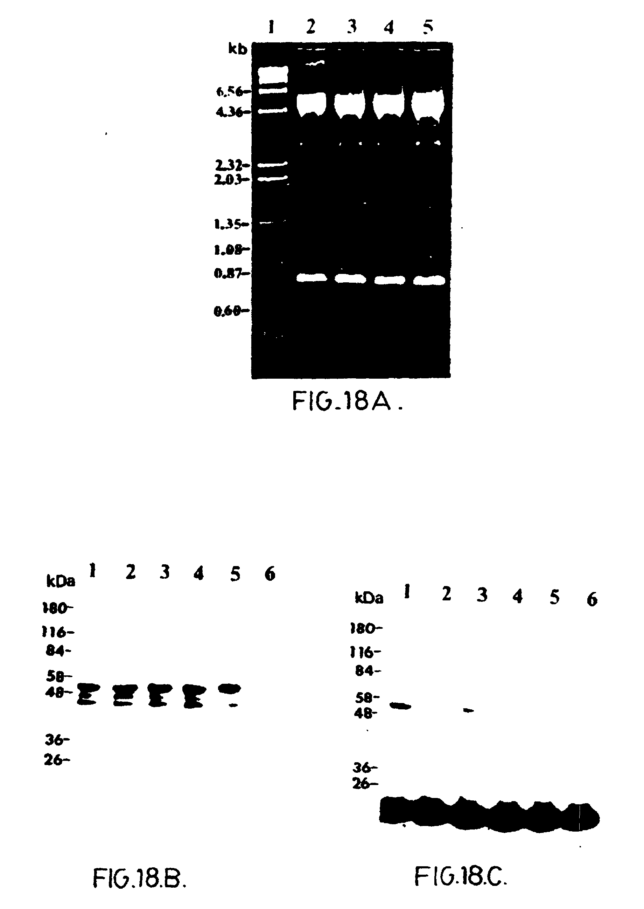

Figure 18A shows DNA of HB101 clones containing:
   - Lane 1:: Normal DNA insert for peptide^{aa423-432};
   - Lane 2:: DNA insert containing Mutation I;
   - Lane 3:: DNA insert containing Mutation II;
   - Lane 4:: DNA insert containing Mutation III;
   - Lane 5:: DNA insert containing Mutation IV;
   - Lane 6:: HB101 with no DNA insert;
Figures 18B and C shows Western blots of fusion proteins from clones of 18A, stained with anti-RHAMM antibody (18B) or with biotin-labelled HA (18C).

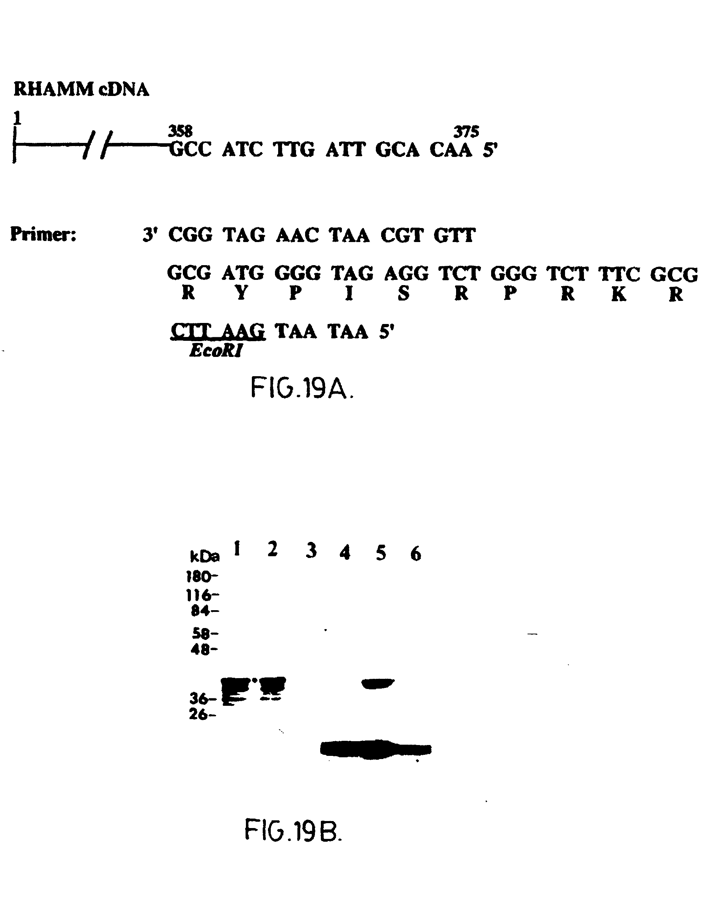

Figure 19A shows the strategy of aligning link protein peptide^{aa316-325} to RHAMM peptide^{aa1-375} (Sequence ID No. of primer : 15).
Figure 19B shows Western blots of fusion proteins, examined for HA-binding.

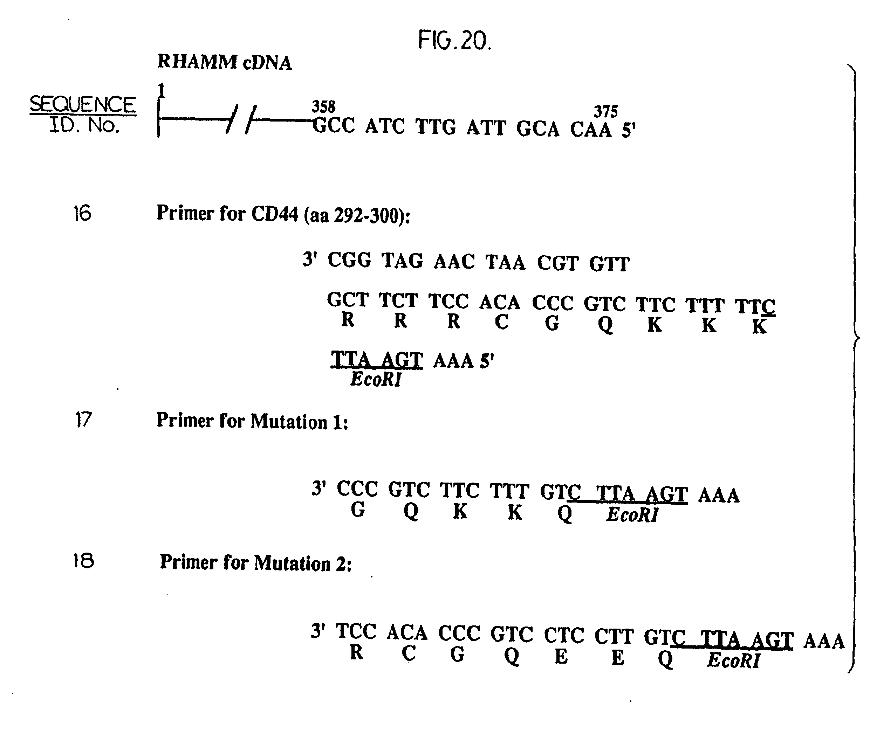

Figure 20 shows strategy for aligning peptide^{aa292-300} of CD44 to RHAMM cDNA, nucleotide 1-375, and for mutating the CD44 peptide.
Figure 21:

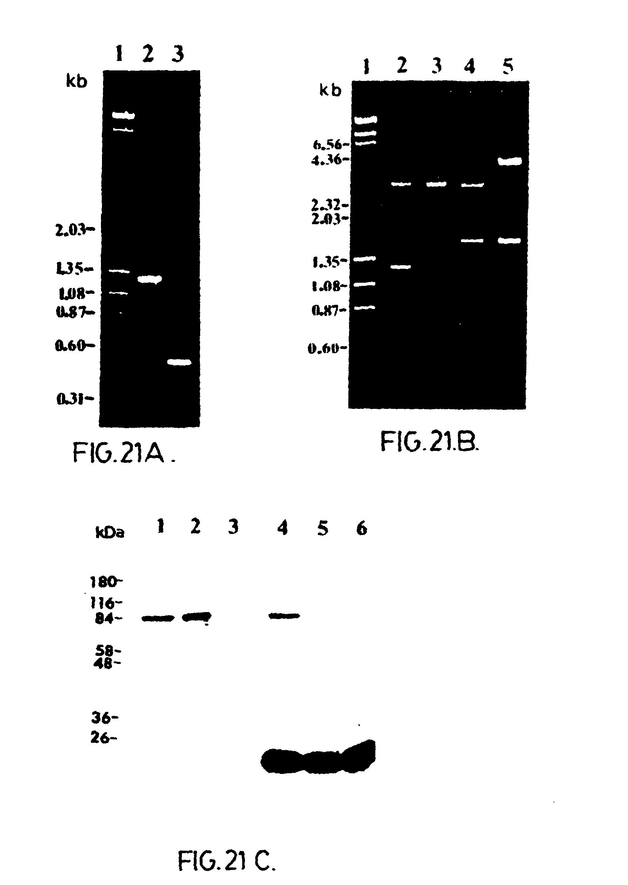

   Figure 21A shows Southern blot of PCR products;
   Figure 21B shows Southern blot of DNA inserts
   Figure 21C shows Western blot of fusion proteins.

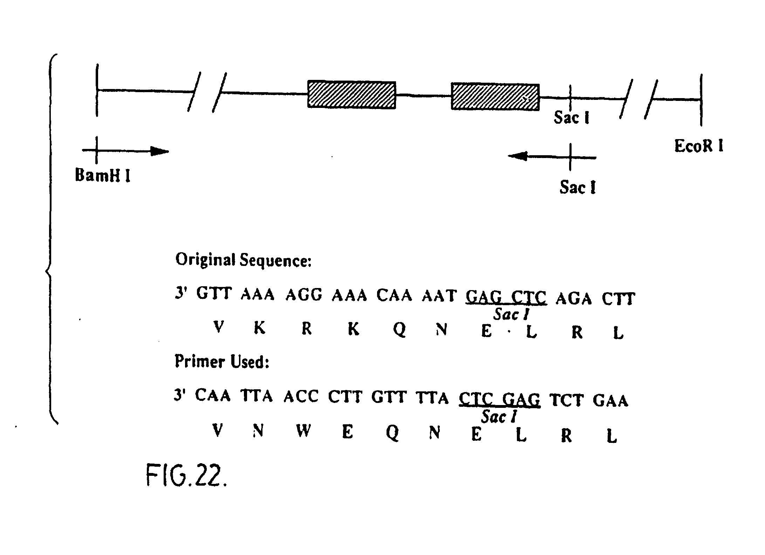

Figure 22 shows strategy for site-directed mutagenesis of peptide^{aa423-432} (K⁴³⁰, R⁴³¹, and K⁴³² mutated to N⁴³⁰, W⁴³¹ and E⁴³² : Sequence O.D. No. of primer : 19).

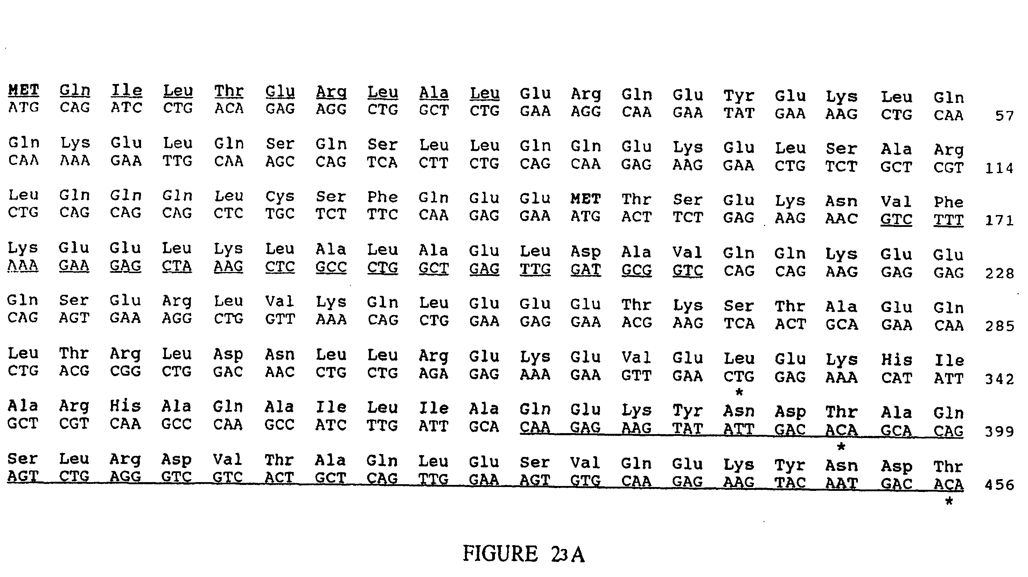

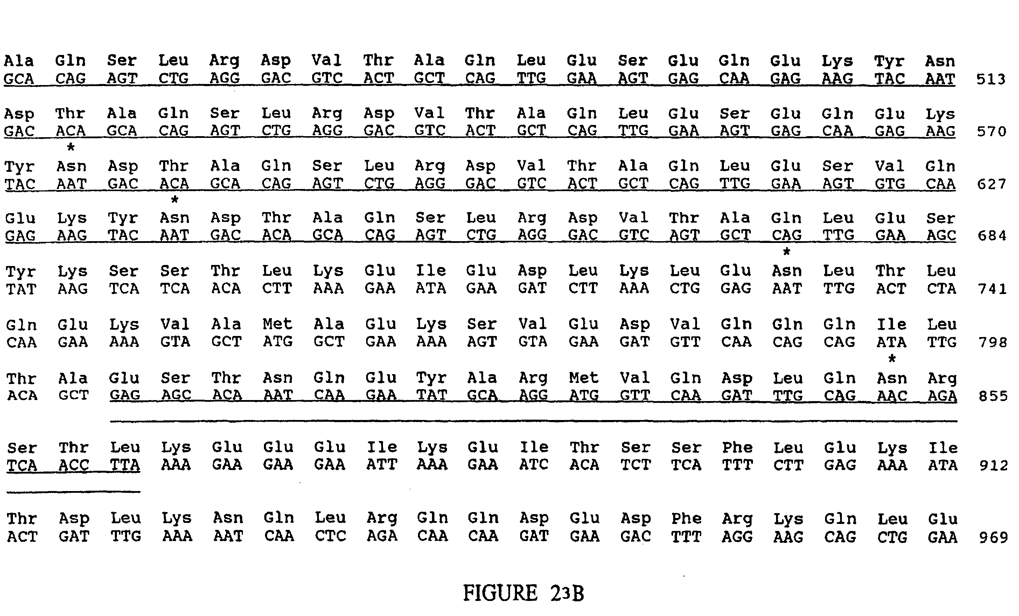

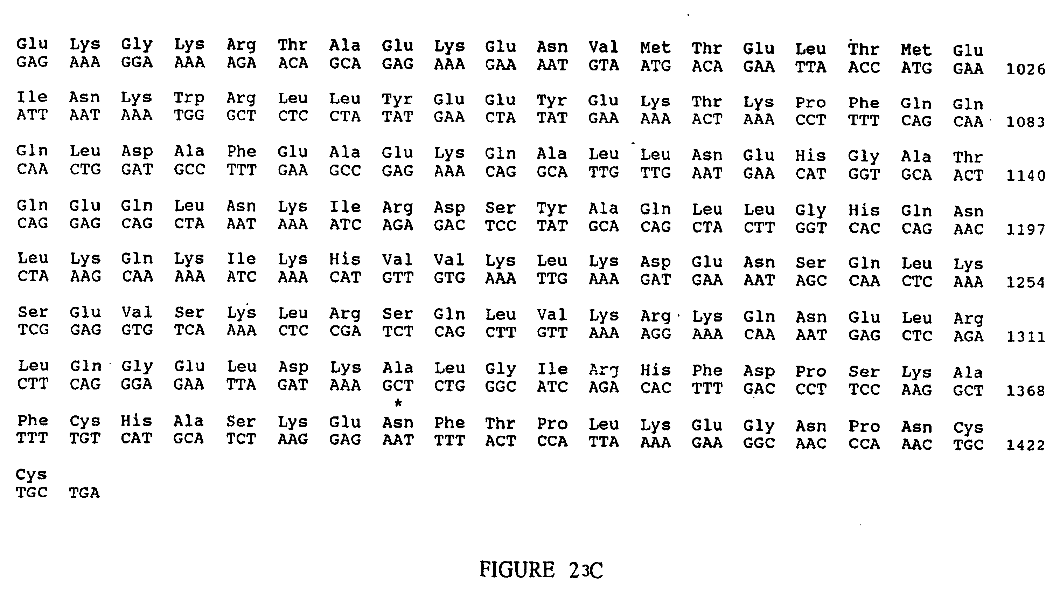

Figures 23A, 23B and 23C show the complete nucleotide sequence (Sequence ID No. 1) and the derived amino acid sequence (Sequence ID No. 2) of a 2.9 Kb cDNA clone encoding the HA receptor of the invention.
Figure 23A shows nucleotides 1 to 456 of the sequence, and corresponding amino acids. Figure 23B shows nucleotides 457 to 969 of the sequence, and corresponding amino acids. Figure 23C shows nucleotides 970 to 1428 of the sequence and corresponding amino acids.

### SUMMARY OF THE INVENTION

The present inventor has isolated and identified a unique HA binding protein which is a component of a novel HARC. It has been shown that this binding protein or HA receptor is involved in cell locomotion or motility. A novel cDNA encoding the HA receptor has been molecularly cloned in the present invention.

The inventor has also identified a novel amino acid sequence motifs which comprises the HA binding regions of the locomotion-associated HA receptor and is also of other extracellular and cell-associated HA binding proteins.

In accordance with one aspect of the invention, an isolated DNA molecule is provided comprising a nucleotide sequence encoding a locomotion-associated hyaluronan binding protein wherein the nuclotide sequence is one of the following sequences:
i) the nucleotide sequence of Figures 23A, 23B and 23C (Sequence ID No. 1);
ii) nucleotide sequence 147-1428 of Figures 23A, 23B and 23C.

In accordance with a further aspect of the invention, an locomotion-associated hyaluronan binding protein is provided comprising the derived amino acid sequence of Figures 23A, 23B and 23C.

In accordance with further aspects of the invention are provided peptides having the formulae:

Lys - Gln - Lys - Ile - Lys - His - Val - Val - Lys;

Lys - Ile - Lys - His - Val - Val - Lys - Leu - Lys;

Lys - Leu - Arg - Ser - Gln - Leu - Val - Lys - Arg; and

Lys - Leu - Arg - Ser - Gln - Leu - Val - Lys - Arg -Lys.

### DETAILED DESCRIPTION OF THE INVENTION

The integrity of complex organisms depends not only upon their resistance to foreign pathogenic organisms but on tight regulation of the differentiation, growth and locomotion of cells comprising tissues. For instance, aberrant regulation of locomotion contributes to host response to tissue injury, expression of birth defects and neoplasia. The molecular mechanisms that regulate cell locomotion are only now being investigated. In the past, attention focused upon the role of adhesive molecules and proteins of the contractile cytoskeleton in motility. Although these components are essential for locomotion, we are now understanding that they are not sufficient.

It is becoming clear that cell locomotion must be initiated and is likely not restrained by physical barriers such as cell-cell contact and basement membranes as was previously supposed. An increasing number of molecules are being described that are potential candidates for the initiation of locomotion. These include autocrine motility factors, growth factors and components of the extracellular matrix. One component of the extracellular matrix that has particularly been implicated in cell locomotion is the glycosaminoglycan hyaluronic acid or hyaluronan. The production of this polysaccharide has been correlated to the migration of embryonic, tumour and lung fibroblasts in a number of laboratories.

Oncogenic transformation by both activated src and ras genes promotes synthesis of HA (29,34) and the growth of many human tumours is accompanied by elevated levels of this glycosaminoglycan in the serum or in tissue surrounding the tumour (25,27). Tumour cells often show increased responsiveness to HA-stimulating factors (4,13) and, recently, the increase in locomotion of tumour cells, particularly ras-transformed cells, has been shown to be mediated by HA (34).

Several investigators have demonstrated that hyaluronan interacts with the cell surface via high affinity specific receptors. A number of receptor candidates have been described but only two cell surface hyaluronan receptors have been molecularly characterized, CD44 (23) and the novel HA receptor to be described. These proteins appear to be unrelated to one another.

Utilizing antibodies specific to the 52, 58 kDa proteins of HARC, the inventor has isolated and characterized a novel 2.9Kb cDNA from a λGT11 3T3 cell cDNA expression library that encodes a 48 or 52 kDa protein, depending upon the initiation codon used. This protein is unique, occurs on the cell surface, is regulated by the mutant H-ras oncogene and mediates locomotion of ras-transformed cells responding to HA.

Antibodies to peptide sequences encoded by the cDNA block the increase in locomotion resulting from induction of the mutant H-ras gene. In a transblot assay, the bacterially expressed protein binds to biotinylated HA. Its unique structure and HA binding properties indicate that it is a new HA receptor. In view of its role in cell locomotion, the protein is referred to as RHAMM, an acronym for Receptor for HA Mediated Motility. RHAMM binds to HA with high affinity (K_{D} = 10⁻⁸M) and specificity.

RHAMM is down-regulated in stationary normal cells and is only expressed in situations where cell motility is required, for example, in wound healing, in response to growth factors and in chemotaxis by white blood cells.

Using monoclonal antibodies specific for RHAMM, it has been demonstrated that it is a tightly regulated protein that is expressed just prior to and immediately following initiation of cell motility in vitro. Furthermore, antibodies to this protein both prevent initiation of locomotion and inhibit rapidly locomoting cells (34,53).

Increased expression of RHAMM has also been observed in several types of tumour cells, including breast tumours, lymphomas and leukemia (96,97).

RHAMM and the HARC complex of which it is a component have been found to be present in all cells examined, in situations where locomotion is involved, including chicken fibroblasts (28) and frog neurites.

These results suggest that the interaction of HA and RHAMM is a universal mechanism for regulating cell locomotion.

As indicated above, RHAMM is a unique protein and its discovery is novel since it has not previously been described at the molecular level. Study of its properties has led to the novel proposal that cell locomotion is initiated and can therefore be molecularly predicted. Further, stimulation appears to occur via messenger mechanisms and not necessarily via alterations of cell adhesion.

The detailed study of the HA binding regions of RHAMM, and the effect of deletions and point mutations on HA binding have enabled the inventor to identify a novel amino acid sequence motif which is not only responsible for HA binding by RHAMM but is present in all other HA binding proteins examined.

The novel amino acid sequence motif associated with HA binding comprises a sequence of 9 or 10 amino acids in which the first and last amino acids are basic amino acids, either lysine or arginine, and the intervening seven or eight amino acids are neutral or basic amino acids. If the two terminal basic amino acids flank an intervening sequence of less than seven or more than eight amino acids, HA binding is lost.

The primary amino acid sequence of the intervening seven or eight amino acids is unimportant, provided no acidic amino acids are included. Inclusion of an acidic amino acid in the intervening sequence either abolishes HA binding or reduces it substantially.

The characterisation of RHAMM and of its constituent HA binding domains and the cloning and sequencing of its cDNA provide tools for controlling or regulating the interaction of HA and RHAMM and therefore for controlling or regulating HA-stimulated locomotion in vertebrate cells, including mammalian cells.

Antibodies to RHAMM or to one or more of its HA binding domains block HA binding and inhibit cell locomotion. Since RHAMM/HA interaction is involved in oncogene-and growth factor mediated-cell locomotion, antibodies to RHAMM, or to variants or fragments thereof which retain HA binding ability, provide means for therapeutic intervention in diseases involving cell locomotion, for example, tumour invasion, birth defects, acute and chronic inflammatory disorders, Altzheimer's and other forms of dementia, including Parkinson's and Huntington's diseases, AIDS, diabetes, auto immune diseases, corneal displasias and hypertrophies, burns, surgical incisions and adhesions, strokes, multiple sclerosis, depression/schizophrenia related to neuronal growth and pain states involving nerve sprouting.

Other situations involving cell locomotion, in which intervention using antibodies to RHAMM or its constituent peptides could be employed, include CNS and spinal cord regeneration, contraception and in vitro fertilisation and embryo development. The inventor has found that antibodies to RHAMM inhibit human sperm motility in vitro and also inhibit fertilisation of hamster ova by human sperm in an in vitro system. Antibodies to RHAMM or to fragments thereof or to its constituent binding peptides may be made by conventional methods known to those skilled in the art.

HA stimulated locomotion may also be controlled by regulation of expression of RHAMM, for example, by use of antisense DNA sequences directed to the RHAMM gene or portions thereof or by gene therapy, for example by homologous recombination using RHAMM cDNA.

RHAMM HA-binding peptides, such as peptide^{aa404-411} and peptide^{aa423-432} may also be employed to interfere with and control RHAMM/HA interaction. These peptides probably act by removing the ligand, HA.

It has also been demonstrated that RHAMM binds to the enzyme responsible for HA synthesis, HA synthase, which is located on the cell surface, and by binding reduces HA production (93). HA-stimulated cell locomotion may therefore be controlled by administration of solubilised RHAMM protein to control HA production.

In view of the tight regulation of RHAMM in normal cells, it provides a useful diagnostic marker for cancer, inflammatory and autoimmune diseases, trauma and recovery from tissue injury associated with vascular disease, toxins or drugs and for predicting birth defects from diagnosable mutations in the RHAMM gene or gene regulating expression of RHAMM.

Insertion of the correct gene would permit correction of diagnosed birth defects associated with abnormalities of the RHAMM gene.

The protein encoded by the DNA of the invention appears to be identical or related to the 58 and 52 kDa proteins of a hyaluronan receptor complex previously reported (29). These proteins have been shown to be involved in locomotion (2,34) and, recently, to also bind to HA (35). Thus, a polyclonal antibody specific to the encoded protein crossreacts with the 52, 58 kDa HARC protein. Conversely, a battery of monoclonal antibodies that are specific for the 52 and 58 kDa HARC proteins crossreact with bacterially expressed RHAMM. These observations combined with the ability of the HARC proteins to reverse the blocking effect of Pab specific to the encoded protein on cell locomotion (Fig. 5), the precise co-localization of the two antigens, their common regulation by the H-ras oncogene, their ability to bind to HA and to mediate ras-regulated locomotion indicate that the gene product isolated corresponds to a component of HARC.

Although the cDNA encoding the 58 kDa protein does not contain a strong signal sequence, the inventor has shown by ultrastructural localisation studies that the protein is located at the cell surface. Although somewhat unusual, a similar lack of signal sequences has been noted for other cell surface receptors including lymphocyte Fc receptor for IgE (10), transferrin receptor (15), liver asialoglyco-protein receptor (9), and the high affinity laminin receptor (18, 36).

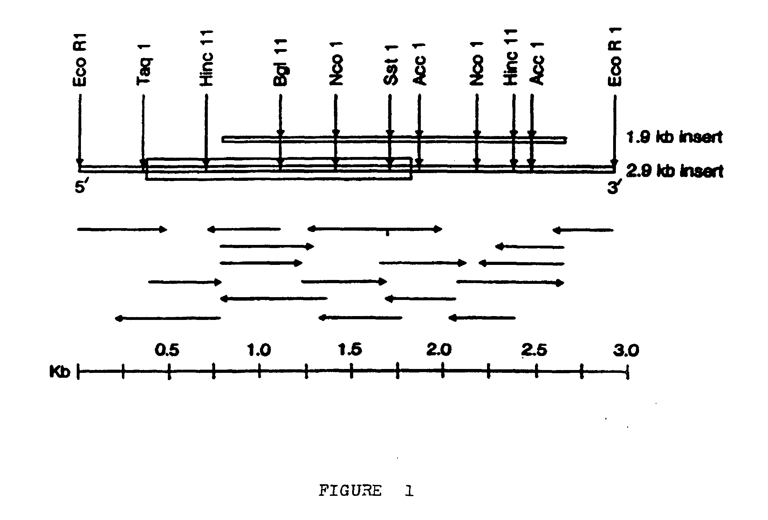

Blocking Pab and Mab antibodies to HARC were prepared and used to screen a λgt11 3T3 cDNA expression library (Clonetech, Palo Alto, CA) as described in Example 1. A restriction map was constructed using the enzymes (U.S. Biochemical Corporation, Cleveland, OH) indicated in the restriction map of Figure 1. The open reading frame of the clones is boxed. The sequencing strategy is shown below the cDNA clones. Both antibodies to peptides I and II encoded in the cDNA (peptide I : nucleotides 372-435; peptide II : nucleotides 804-864), and a radiolabeled ACC I fragment of the 1.9 kb insert were used to isolate the 2.9 complete cDNA. The amino acid sequences are shown above the DNA sequences in Figure 1. Two possible initiation codons are indicated with highlighted letters. The cDNA encodes a 48 or 52 kDa protein, depending on which initiation codon is used. Potential N-glycosylation sites are marked with asterisks and possible signal sequences are underlined with broken lines. The stop codon is indicated by highlighted letters.

Restriction mapping and sequencing of the 2.9 Kb insert demonstrated that it encodes a complete protein and contains the original 1.9 Kb cDNA sequence in its central region (Figure 1). The sequence is unique and does not bear significant homology to other proteins registered in NERB or EMBL data banks or to factors known to be involved in ras regulated locomotion (14). Like previously characterized proteins such as p53 (37), it contains two possible initiation codons, encoding proteins of either 52.2 kDa or 46.7 kDa respectively (Fig. 1B). The encoded protein is rich in glutamic acid, lysine, glutamine and leucine. It has a Pi of 5.2, is hydrophilic and most of the polypeptide is predicted to occur as an alpha helix by Chou Fasman analyses (5). The most notable feature of the deduced sequence is a 21 amino acid stretch (which corresponds to Peptide I,) that is repeated 5 times near the N-terminus. The predicted protein contains 8 putative N-glycosylation sites, 5 of which are concentrated within the repeated motif. The protein also contains clusters of positively charged amino acids throughout the open reading frame. It does not encode a hydrophobic sequence long enough to span the plasma membrane and possible signal sequences following either initiation codon are weak (Fig. 1).

### The Encoded Protein Occurs at the Cell Surface

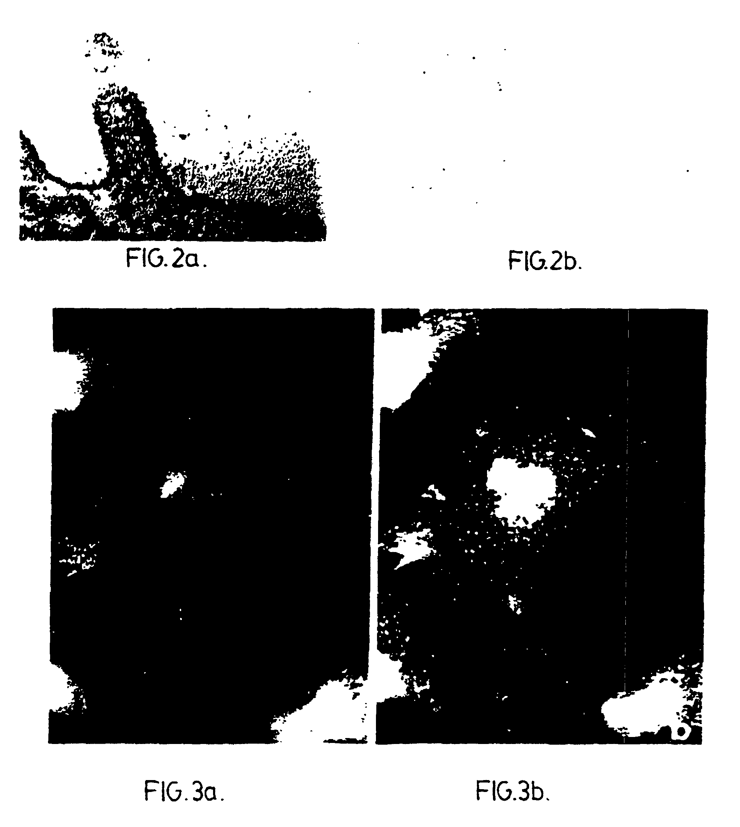

In spite of an absence of a consensus, hydrophobic region encoded in the cDNA, the protein occurs at the cell surface as demonstrated by ultrastructural localization of immunogold to the cell membrane (Fig. 2), and by live cell immunofluorescence using Pab to Peptide II. At the light microscope level, the encoded protein is seen to strikingly accumulate in the ruffles and processes of H-ras transformed cells (Fig. 3). It also occurs intracellularly in the perinuclear region (data not shown). This distribution is typical of molecules that regulate cell locomotion (1,14). It is further noted that Pab I and II co-localize precisely with Mab to the 58 kDa HARC protein.

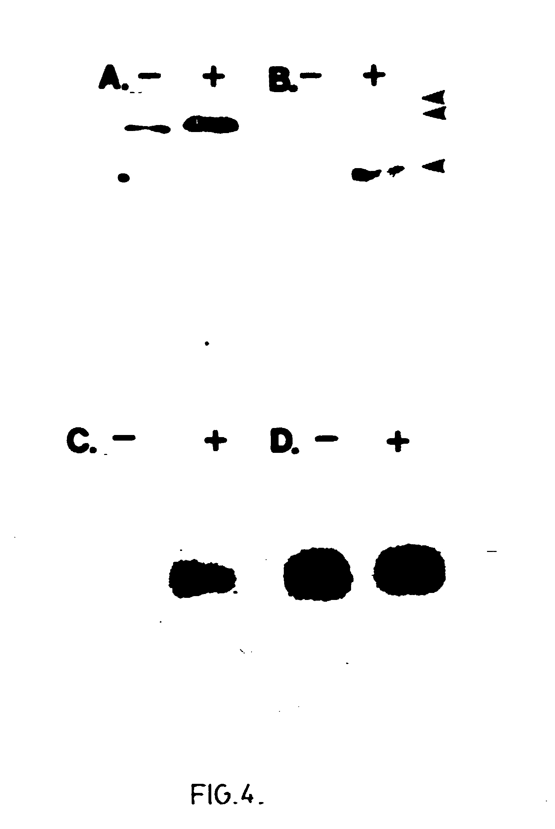

Zinc sulphate induction of a metallothionein-regulated mutant H ras gene transferred into 10T½ fibroblasts (26) gave increased expression of a 58 kDa protein detected by Pab to Peptide II, as seen in Figure 4A, as well as increased expression of p21 Ras proteins (Figure 4B). These results indicate that RHAMM expression is regulated by the mutant H-ras gene. The induction of the mutant ras gene gave increased expression of a 5.2 kb mRNA transcript, as seen in Figure 4C.

### Antibodies to Peptides Encoded in the RHAMM cDNA Block Locomotion

Direct evidence for a role of the encoded protein in H-ras regulated cell locomotion was demonstrated in experiments designed to test whether antibodies to the encoded protein inhibited cell locomotion. As noted previously (34), induction of the mutant H-ras gene with zinc sulfate activated an HA-dependent motility mechanism in mutant H-ras transfected fibroblasts (Fig. 5). Antibodies to peptide II specifically inhibited ras regulated locomotion (Fig. 5). As well, transfection of a RHAMM genomic clone into 10T½ fibroblasts resulted in overexpression of RHAMM at the cell surface and an 8-fold increase in the rate of cell locomotion (data not shown).

### Bacterially Expressed RHAMM binds to biotinylated HA

A 1.3 Kb insert encoding RHAMM (insert containing the open reading frame from the second initiation codon) was expressed in E. coli, as described in Example 1. RHAMM was identified on immunoblots, using Mab 3T3-5, specific for the 52,58 kDa HARC proteins, as a 70 kDa protein, as seen in Figure 6, Lane D. The expressed protein specifically bound to biotinylated HA (Lane A) and the binding was competed with by excess unlabelled HA (Lane B). Bacterial lysates from bacteria containing plasmids without the insert did not show HA binding (Lane C).

In transblot immunoassays utilising purified HARC proteins as substrate, it was shown that Pab to peptide I cross-reacted with the 58 and 52 kDa proteins of HARC (Figure 7, Panel C). The locomotion blocking antibody Mab 3T3-5 also cross-reacted with these proteins (Figure 7, Panel B). These results show the antigenic relationship between the protein encoded by the cDNA of the invention and the 52,58 kDa HARC proteins, which are the HA binding proteins of the complex (35).

A complete RHAMM cDNA (1.43 kb) was expressed as a fusion protein with pGEX-2T in E. coli HB101 and was shown to bind specifically to biotin-labelled HA and to antibodies specific for RHAMM in a transblot assay, as seen in Figure 8, and to HA-Sepharose. The complete cDNA was truncated with restriction endonucleases from the 3' end resulting in 1.30 kb, 1.02 kb, 0.71 kb and 0.41 kb cDNAs which were then expressed in HB101. The truncation strategy is shown in Figure 9. Only the fusion peptide expressed from the complete cDNA and the 1.30 kb cDNA bound to HA, as seen in Lanes 6 and 5, Figure 10B, indicating that the region located between 1.02 kb to 1.30 kb of RHAMM cDNA is critical for recognition of this glycosaminoglycan. Deletion of 114 bases in this region virtually eliminated HA binding activity thus defining the major glycosaminoglycan binding region to amino acids 400-434 located near the carboxy terminus of RHAMM, as seen in Figures 12A and B. Two domains containing clusters of basic amino acids were identified within this region, peptide ^{aa 401-411} and peptide ^{aa423-432.} Synthetic peptides corresponding to these two domains both inhibited HA binding to the complete 1.43 kb expressed GST-RHAMM fusion protein, and also directly bound to HA-Sepharose, as seen in Figure 13A and B. Random peptides and peptides mimicking other regions of RHAMM did not inhibit HA:RHAMM interactions and bound weakly to HA-Sepharose. Oligonucleotides encoding either of these two peptides were linked to the N-terminal 0.71 kb of RHAMM which encoded a peptide that did not contain HA binding activity. Fusion proteins containing either of these recombinant peptides acquired HA binding activity as assessed with a transblot assay, shown in Figure 14B.

The inventor has shown by study of the HA binding domains of RHAMM that the structural features required for HA binding comprise a novel amino acid sequence motif, a sequence of nine or ten amino acids, with a basic amino acid in each terminal position and the intervening positions being either basic or neutral amino acids, but not acidic amino acids.

Three such motifs occur in RHAMM, peptide^{aa401-410}, peptide^{aa402-411} and peptide^{aa423-431}.

The presence of further basic amino acids, in addition to the terminal positions, either within the motif, or adjacent to the motif, will give increased binding affinity to HA.

The inventor's studies indicated that the positions of the basic amino acids in the HA binding domains of RHAMM are important.

Since HA is composed of repeating disaccharide units of N-acetyl glucosamine and D-glucuronic acid, it was predicted that each tetra- to hexa- saccharide unit of HA was the functional unit for HA to bind to receptors spanning at least two carboxyl groups of HA. Each HA binding domain in previous results had approximately 10 amino acid residues and both of them had α-helical folding, fitting with this model (91). The distance of each disaccharide unit (or distance of two negative charges) spaces approximately 9 amino acids. Thus, the presence of basic amino acids at 1 and 9 or 10 positions will increase the binding affinity to HA since the interaction is ion dependent. Both HA binding domains of RHAMM have basic amino acids at 1 and 9 positions (two basic amino acids spaced apart by 7 amino acids). The random peptides shown to have much lower affinity to HA-Sepharose had no basic amino acids at positions 1 and 9.

RHAMM has, however, five domains in which two basic amino acids are separated by seven amino acid residues. These are peptide^{aa54-62}, peptide^{aa90-98}, peptide^{aa98-106}, peptide^{aa227-} ²³⁵, and peptide^{aa320-328} (53). These domains do not have the ability to bind HA since the deletion of the two HA binding domains, peptide^{aa401-411} and peptide^{aa423-432}, abolished the HA binding activity of the remaining polypeptide (Fig. 12B, lane 3). It was noted that acidic amino acids were present between the two basic amino acids in all of these five domains that did not bind to HA. This suggested that acidic amino acids were not compatible with HA binding, since charges are the basis of the interaction.

The inventor has shown that deletion of peptide^{aa423-432} along with partial deletion of peptide^{aa401-411} from RHAMM (Figure 22) completely destroyed the ability of the receptor to bind to HA, indicating that other regions of the protein do not bind to HA, even though other domains have two basic amino acids separated by seven intervening amino acid residues.

To further test the inventor's HA-binding motif model, a number of synthetic peptides were prepared and their HA-binding ability was determined using HA-sepharose. A tyrosine residue was added to one end of each synthetic peptide for convenient spectrophotometric quantitation.

The results are shown in Figure 16. Peptide B (Y K Q K I K H V V L L L) had low ability to bind to HA-sepharose; this peptide has a basic amino acid at position 1 but none at position 9 or 10. Peptide A (Y K Q K I K H D D K L K) also had low ability to bind to HA-sepharose; this peptide has acidic amino acids between the basic amino acids at positions 1 and 9. Peptide D (Y R V R G V L G K R R) had the highest ability to bind to HA-Sepharose; this peptide contains both requirements for HA binding, basic amino acids at positions 1 and 9 and no intervening acid amino acids. The ability of peptide C (Y K Q K I K H V V L K L) to bind to HA-Sepharose was quite high, probably due to the presence of basic amino acids at positions 1 and 10.

The importance of the key basic amino acids for HA binding to RHAMM was pinpointed by point mutation of these amino acids, with accompanying loss of HA binding.

Figure 17A shows the strategy of site-directed mutations of peptide^{aa423-432} employed. Panel B lists the resulting mutated amino acid sequences.

DNA inserts coding for peptide^{aa423-432} or for a mutation were inserted into pGEX-2T and transformed into HB101. Fusion proteins expressed from these DNA inserts were examined for HA binding, as shown in Figure 18.

The results show that the HA-binding ability of the first 3 site-directed mutated samples was crippled (Figure 18C, lane 2-4, mutations I-III), and that of the last site-directed mutated sample was completely destroyed (lane 5, mutation IV).

The results indicate that change of neutral amino acid V to an acidic amino acid D (Fig. 18C, lane 3) slightly decreased its HA-binding ability. The change of a basic amino acid to a neutral amino acid decreased HA-binding ability seriously (Fig. 18C, lanes 2 and 4). The change of KRK to HHH completely destroyed HA-binding ability (Fig. 18C, lane 5). These results demonstrate that basic amino acids are crucial in HA-binding motifs. Acidic amino acids negatively affect HA binding ability. His is apparently not important in HA binding; substitution of His for Arg or Lys abolishes HA binding.

Studies of HA binding proteins other than RHAMM have indicated that sequences containing clusters of basic amino acids are involved in HA binding, eg. tandem repeat sequence in cartilage proteoglycan (83,84) and rat link protein (48,7).

Examination of the published amino acid sequences of various HA-binding proteins, however, disclosed no homology of amino acid sequence which could be linked to HA binding.

The inventor has recognised the novel amino acid sequence motif described above as the binding domains of RHAMM and has also identified this unique motif in all reported sequences of HA binding proteins.

These findings are set out in Table 1. In Section II of Table 1 are listed the binding regions of various HA-binding proteins as reported in the literature, along with the primary amino acid sequence of the novel HA-binding sequence motif of the invention identified within these binding regions.

As shown in Section II of Table 1, link protein (8), versican (92), hyaluronidase (HAase, 98) and aggrecan (7) all contain one or more HA-binding motifs, with either lysine or arginine in positions 1 and 9.

The inventor has identified the unique HA binding motif at peptide^{aa71-79} of the G1 domain of rat cartilage proteoglycan core protein, the G1 domain having been shown to be an HA binding domain by Doege et al. (7). The G2 domain of rat cartilage proteoglycan core protein has been shown not to bind HA (7), although containing similar amounts of basic amino acids (9% in G1 and 8% in G2). The recognition by the inventors of the HA binding motif explains the lack of HA binding of the G2 domain as the critical motif does not occur in G2.

The inventor has performed genetic manipulations on link protein and on CD44 and confirmed the HA-binding ability of the putative binding domains identified by recognition of the novel sequence motif of the invention. These binding sequences are shown in Table 1, Section I.

Link protein contains an HA-binding motif at peptide^{aa316-325} and at peptide^{aa103-112.} Attachment of link protein peptide^{aa316-325} to a part of the RHAMM polypeptide that does not bind HA, as described in Example 3, conferred HA-binding properties on that peptide.

The results are shown in Figure 19B.

A tandem repeat loop of link protein has been demonstrated to be an HA binding domain by solid-phase assay using immobilised HA and synthetic peptides (8). The inventors have identified the unique HA binding motif in the tandem repeat loop of link protein, at peptide^{aa316-325} and confirmed by genetic manipulation that this sequence is an HA binding domain (Figure 21).

Genetic manipulation of CD44 was also carried out. A domain R R R C G Q K K K (peptide^{aa292-300}) of CD44 was aligned by PCR technique to the 5' region of RHAMM cDNA (nucleotide 1-375) that encodes a non-HA-binding polypeptide. The strategy is shown in Figure 20. This CD44 domain was then mutated to R R R C G Q K K Q using primer for mutation 1 and to R R R C G Q E E Q using primer for Mutation 2. Fusion proteins were prepared from selected clones and subjected to Western blot assays stained with anti-RHAMM antibody or biotinylated HA (data not shown).

When peptide^{aa292-300} was linked to the N-terminal domain of RHAMM, the fusion protein bound HA. When amino acid 294 or 295 was mutated, the HA-binding ability of the fusion protein was destroyed.

### EXAMPLES

### EXAMPLE 1

The following description contains details of methods used and results obtained by the inventor in the isolation and characterization of RHAMM.

The following abbreviations are used:
GAG, glycosaminoglycan; Pab, polyclonal antibody; Mab, monoclonal antibody; SDS, sodium dodecyl sulfate; HRP, horse radish peroxidase; TBS, tris buffer saline; TRIS, Trizma HCL.

### Antibodies

Polyclonal (Pab) and monoclonal (Mab) antibodies to HARC were prepared as described previously (28,29). All antibodies were purified by affinity chromatography on HARC-Sepharose and/or Protein A-Sepharose. Mab 3T3-5, which blocks fibroblast locomotion, was originally used to screen the cDNA library. Mab 3T3-5 is shown here to recognize 52-58 kDa proteins in SDS-immunoblots of lysates of ras transformed fibroblasts and has previously been shown to block HA-promoted locomotion of these fibroblasts (29). Mab 3T3-7, which was used for immunofluorescence, reacts specifically with the 52-58 kDa proteins of HARC, as shown here by SDS immunoblot analyses, but does not block HA-promoted locomotion (29). Synthetic peptides mimicking two amino acid sequences in the deduced sequence [Peptide I : nucleotide sequence 372-435 of Figure 1 (corresponding to a portion of the repeated sequence) and Peptide II : nucleotide sequence 804-864 of Figure 1] were produced by the Peptide Analysis Core of the University of Alabama Cancer Center (Birmingham). The peptides were purified by HPLC and characterized by amino acid composition analyses. Antibodies to the peptides were raised in rabbits as described (28, 29). These antibodies were purified by affinity chromatography on columns of the appropriate peptides coupled to CNBr-activated Sepharose and the specificity of purified anti-peptide antibodies to the respective peptide was confirmed by dot blot assays. Preimmune antibodies were obtained from each rabbit or mouse prior to immunization, purified as described above and used as controls.

### Cloning and DNA sequencing

A 3T3 cDNA library in λgt11 was obtained from Clontech (Palo Alto, CA) and initially screened with both Pab and Mab (3T3-3) to HARC. One clone, which gave a positive signal upon repeated screening with the antibodies, had a 1.9 kb insert that was subcloned into pUC18 and M13.

Sequencing of the insert revealed an open reading frame corresponding to a 340 amino acid residues of a C-terminal protein segment that did not include an initiation codon (Fig. 1). Additional clones coding for the same protein were isolated by rescreening the library with a radiolabelled Acc 1 restriction fragment of the 1.9 cDNA and with Pab to peptide sequences (peptide I and II, Fig. 1) encoded in the insert. A 2.9 Kb clone was positive in both screenings and was further characterized (Figure 1).

The restriction map was constructed using the enzymes indicated in Figure 1 (U.S. Biochemical Corporation, Cleveland, OH). Nucleotide sequences were determined by the dideoxy chain termination method using Sequenase I and Sequenase II kits (U.S. Biochemical Corporation, Cleveland, OH). Digestion of the insert with Acc 1 yielded a fragment which was radiolabelled with a Random priming kit (BRL, Bethesda, MD) and was used to rescreen the cDNA library at high stringency.
The inserts were isolated after digestion of the λphage with Eco R1 and ligated into pUC18 and M13 for sequencing. Sequence analysis and comparisons were conducted using IBI/Puskell sequence analysis software and NBRF database (version 18, 4). Additional homology searches were conducted with NBRF (6/90) and EMBL (5/90) using FASTA and word search programs in GCG software.

### Ultrastructural Localization of Encoded Protein

For ultrastructural localization of the encoded protein, H-ras expressing tumor cell monolayers were processed with A) Pab to Peptide II (0.5 µg/ml) or (B) preimmune IgG, (0.5 µg/ml) followed by gold conjugated goat anti-rabbit IgG (5 nm, Sigma Chemicals, St. Louis, MO). Antibody localized gold particles to the cell surface (magnification 30,780 fold). Sections incubated with pre-immune sera did not bind particles (magnification 30,780 fold) (Fig. 5).

### Immunofluorescent Localization of Encoded Protein Relative to HARC Proteins

Double immunofluorescence studies of zinc-induced fibroblasts were performed as described (28,32) using (a) Mab 3T3-5 which specifically reacted with 52-58 kDa proteins of HARC and (b) Pab to Peptide II (nucleotide 5 sequence 804-864). The Mab was detected with FITC-anti-mouse IgG and the Pab was detected with RITC-anti-rabbit IgG.

Secondary antibodies were rhodamine labelled goat anti-mouse IgG (to detect Mab 3T3-7) and fluorescein labelled goat anti-rabbit IgG (to detect Pab to Pep. 1) and were purchased from sigma Chemicals (St. Louis, MO). Processed monolayers were examined with a Zeiss IM35 equipped with epifluorescence utilizing non-overlapping filters of 510-560 mm (for rhodamine) and 450-490 nm (for fluoresence). Lack of bleedthrough was confirmed by examination of single immunofluorescence samples with both filters (data not shown).

Both antibodies were strikingly localized in the ruffles and processes of zinc-sulfate induced fibroblasts as seen in Figure 3. Pre-immune sera showed no immunofluorescence (data not shown). Magnification 6000 fold.

### Immunocytochemistry and Electron Microscopy

Fibroblasts transfected with the mutant ras gene were grown overnight on glass coverslips that had been treated with Teflon and baked at 250°C for 30 min. Cells were incubated with primary antibodies (0.5 µg/ml, Pab to Pep I) or preimmune IgG (0-5 µg/ml) for 1 hr and then incubated with gold-conjugated goat anti-rabbit IgG (5 nm, Sigma Chemicals, St. Louis, MO), fixed with 0.1% glutaraldehyde in PBS and postfixed in 4% osmium tetroxide (31,33). Cultures were dehydrated through alcohol series, infiltrated with Spurr resin (1:1 resin:alcohol for 2 h at 37°C) and embedded in 100% Spurr for 12 h at 60°C. The glass coverslips were removed from the hardened resin and ultra-thin sections were obtained with a diamond knife (IVIC, 2.0 mm). Sections were examined with a Phillips 300 transmission electron microscope.

### Expression of encoded protein is regulated by the mutant H-ras oncogene.

Cell lysates were prepared from buffer (-) and zinc sulfate treated (+) 10T½ fibroblasts transfected with mutant H-ras under the control of a metallothionein promoter (26) as described previously (34). Proteins were fractionated on SDS-PAGE (12.5%) transferred to nitrocellulose and probed with the primary antibodies, Pab to Peptide II (nucleotide sequence 804-864, 0.1 µg IgG/ml) or RAS-10 antibody, the results being shown in Figure 4, Panels A and B respectively.

### Isolation of RNA and Northern Assays

Confluent cultures of 212 cells were exposed to zinc sulphate or to buffer alone (34) for 24 hours. Total RNA was extracted according to Choy et al. (6). 60 µg of RNA was electrophoresed on 1% agarose gels and transblotted onto Nytran Nylon membranes. The mRNA transcript for the encoded protein was detected using ³²P-labelled 1.2 kb fragment of the cDNA that contained the open reading frame, the results being shown in Figure 4C. Blots were reprobed with ³²P-GAPDH to control for RNA loading and the results were shown in Figure 4D.

### Locomotion Assays

A 10T½ cell line containing an inducible EJ-ras metallothionein-neomycin hybrid gene (26) was used for quantitating the effect of both Pab to peptide II and Mab to HARC on cell locomotion in response to HA as previously described (34). Antibodies (Mab 3T3.5, 1 µg/ml, Pab Peptide II, 1 µg/ml) were added to zinc induced (24 hr), HA treated cells, ½ hr before filming, then cells were filmed by video timelapse for a total of 2 hrs. To assess specific effects of the antibodies, they were preincubated for 1 hr at 4°C with 50 µg of HARC proteins prior to their addition to control locomotion assays. Addition of these proteins by themselves had no effect on cell locomotion (results not shown). Data were analyzed with a Zeiss Dynacell program that utilized Fourier analysis of cell locomotion to derive a cell motility index (17).

The effect of non-immune sera, Mab 3T3-5 to HARC (1 µg/ml), and Pab to Peptide II (nucleotide sequence 804-964, 1 µg/ml) on HA-promoted locomotion was analyzed. Both Mab 3T3-5 to HARC and Pab to Peptide II inhibited cell locomotion in response to RA. This effect was reversed by the addition of excess HARC proteins.
Non-immune sera had no effect on locomotion relative to controls. Values represent the mean + S.E.M. N=50 cells. The results are shown in Figure 5.

### Expression of RHAMM in Bacteria

Oligoprimers corresponding to the second methionine were prepared and the open reading frame from this start codon was amplified with PCR. The second initiation codon was chosen since the size of the protein generated by N-glycanase digestion of the 58 kDa protein (data not shown) closely matched that predicted using the second initiation codon (35). PCR generated a 1.3 Kb DNA fragment that was cloned into the PGEX-2 T vector (22).
The vector was used to transform JM101 E.Coli and expression was induced by adding 0.2mM isopropyl-beta-D-thiogalactopyranoside (IPTG) as described (22). Isolated bacterial cells were sonicated in a 0.5 mTris (pH 8.0) buffer containing 4M urea, 1% triton x-100 and 1mM EDTA.
The urea was removed by a two step dialysis procedure. The sonicate was initially dialized for 6 hours at 4°C into phosphate buffered saline (pH 7.4) containing 1% triton. The soluble material was applied to a glutathione-agarose affinity gel as described (22) and bound protein was eluted with 0.2 mM glutathione in 0.05 M, Tris (pH 8.0). The purified, expressed protein was then electrophoresed on 12.5% SDS-PAGE.

### Hyaluronan Binding Assay

Lysates from bacteria either with plasmids containing an insert encoding RHAMM or with plasmids without the insert were prepared by sonication.
Twenty-five micrograms of lysate protein were electrophoresed on 10% SDS-PAGE and transblotted onto nitrocellulose membranes. Additional binding sites were blocked with 5% defatted milk and incubated for 1 hour with or without biotinylated HA prepared as described (35). The membrane was washed for 1 hour in Tris-buffered saline containing 0.05% TWEEN then incubated with streptavidin-HRP (1:1000 dilution Sigma Chemicals). The bound streptavidin was visualized with chemiluminescence (Amersham ELL) according to instructions.

### Bacterially expressed RHAMM binds to biotinylated HA

Bacterial plasmids containing a 1.3 Kb insert encoding RHAMM (Figure 6, Panels A,B,D) or no insert (Figure 6, Panel C) were sonicated, electrophoresed on SDS-PAGE, trans blotted onto nitrocellulose and the remaining protein binding sites blocked with casein as described above. Biotinylated HA was added to the blot in the absence (A) or presence of 100-fold excess unlabelled HA (B). Bound HA was then detected with streptavidin-HRP and visualized with chemiluminescence. Bacterial lysates containing the 1.3 Kb insert were incubated with monoclonal antibody 3T3-5 (D) to the 58, 52 kDa HARC proteins and visualized as described. These results (Figure 4) show that HA bound to the expressed protein and binding was competitive with unlabelled HA. MAB 3T3-5, that blocks locomotion, specifically recognized the expressed protein. The molecular weight standards are marked with arrow heads and include α2 macroglobulin (180 kDa); B-galactosidase (116 kDa); Fructose-6-phosphate kinase (84 kDa); pyruvate kinase (58 kDa) and fumarase (48.5 kDa).

### SDS-Immunoblots

Immunoblot assays were conducted on isolated soluble HARC proteins (29) or cell lysates prepared from H-ras transfected fibroblasts exposed to either zinc sulphate or buffer alone for 24 hours. The latter were prepared with lysis buffer containing 25 mM Tris, 0.1% SDS, 1% Triton X-100, 1% sodium deoxycholate, 0.15 M NaCl, 1 mM EDTA and protease inhibitors (29). Proteins were fractionated by SDS-PAGE on 12.5% polyacrylamide gels (29) and transferred to nitrocellulose membranes (Biorad, Rockville Center, NY). Additional protein binding sites were blocked with 0.5% defatted milk in Tris buffered saline (TBS, 34) and the membrane was incubated with primary antibodies (1 µg IgG/ml of TBS containing 0.5% defatted milk) for 12 hr at 4°C on a gyratory shaker (Stovall, Greensboro, NC). The monoclonal antibody to p2l ras (pan, ras-10) was purchased from Dupont. Primary antibodies were detected by chemiluminescence as described in instructions (ECL, Amersham). Briefly, the washed membranes were incubated with horseradish peroxidase (HRP) labelled IgG secondary goat antibody for 20 min to 1 hr at room temperature. After washing, the membranes were developed with peracid followed by luminol and an enhancer. The oxiacid product was detected by immediately exposing with Kodac X-Omat Xac-5 film.

### Immunoreactivity of anti-peptide antibody to the encoded Protein

HARC proteins were purified from spent culture media as described (29) then fractionated by SDS-PAGE and transblotted onto nitrocellulose filters. HARC proteins were visualized with India ink (Figure 7, Panel A) and have MWe of 72, 68, 58 and 52 kDa (arrows). The 52-58 kDa proteins reacted specifically with B) Mab 3T3-5 (blocker of locomotion) to HARC (Panel B), and Pab to Peptide I, nucleotide sequence 372-435, (Panel C). Molecular weight protein standards are marked by arrow-heads and include from the top of the gel, phosphorylase b (97.4 kDa), bovine serum albumin (68 kDa), ovalbumin (43 kDa) and trypsin inhibitor (21.5 kDa).

### Example 2

### Construction of RHAMM cDNA

The RHAMM cDNA reading frame was amplified with Polymerase Chain Reaction (PCR) (61) using two oligonucleotides as primers: one complimentary to the translation initiation region (nucleotide 1-22) creating a BamHI site linked to nucleotide 1; the other complementary to the region 280 bases after the translation stop codon (nucleotide 1685-1706) creating an EcoRI site linked to nucleotide 1706. The full length cDNA inserted in Bluescript (Stratagene, La Jolla, CA) was used as a template. The reaction was carried out at 94°C (30 sec), 54°C (30 sec), and 72°C (90 sec), for a total of 25 cycles (61). The PCR product (1.7 Kb) was digested with proteinase K (50 µg/ml) in 0.5% SDS at 37°C for 30 min. DNA was extracted from the mixture with an equal volume of phenol:chloroform (1:1), an equal volume of chloroform, then ethanol precipitated (61). The PCR product was doubly digested with EcoRI and BamHI and purified by agarose gel electrophoresis. The EcoRI-BamHI DNA fragment was ligated to an EcoRI and BamHI opened pGEX-2T plasmid (62,22, Pharmacia).

The above ligation mixture was transformed into competent E. coli HB101 cells as described (61,63). Colonies were streaked on a master LB/amp agar plate and a replica was prepared by overlaying a Nylon Hybond N1' membrane on the LB/amp agar plate. Both plates were incubated at 37°C for 3 h. The master plate was kept at 4°C. The Hybond N' membrane was screened with RHAMM cDNA as a probe (53). The hybridization was carried out at 65°C in a solution containing 6 x SSC, 5 x Denhardt's solution, 0.5% SDS, and 20 µg denatured salmon sperm DNA per ml hybridization solution overnight as described (22). The membrane was washed at 65°C with 2 x SSC, I x SSC, 0.1. x SSC (30 min for each washing) and exposed to Kodak X-Omat film as described (22). Positive colonies, which indicated the presence of a RHAMM cDNA insert, were recovered from the master plate, and the presence of a correct RHAMM cDNA insert was confirmed with appropriate restriction endonuclease digestion and aqarose gel electrophoresis to determine the size of the insert.

### Fusion Protein Preparation

Colonies of pGEX-2T plasmids containing RHAMM cDNA were grown in 5 ml LB/amp medium at 37°C overnight. 0.1 mM isopropylthio-β-D-galactoside (IPTG) (100 mM stock solution in water) was added to the cultures (0.5 mg protein/ml) to induce the biosynthesis of fusion proteins (63). The cultures were grown for 90 min in the presence of this inducer. The cells were harvested by centrifugation and lysed in 1 ml 50 mM Tris-Cl, pH 8.0, containing 2 M Urea and 1% Triton X-100 (53). Cells were further disrupted by sonication and centrifuged at 15,000 xg for 20 min to separate soluble from insoluble fractions. The supernatants that contained the GST-RHAMM fusion protein were recovered and used for Western blot assay.

### Identification of Fusion Protein

GST-RHAMM fusion protein was prepared from lysates of colonies of HB101 containing the RHAMM cDNA and electrophoresed on SDS-PAGE as an 83 kDa protein (Fig. 8). On Western blot assay, this fusion protein reacted with i) polyclonal antibodies to a peptide encoded in RHAMM cDNA (peptide¹²⁵⁻¹⁴⁵) (53) (Fig. 8, lane 2), ii) a monoclonal antibody specific to RHAMM (Fig. 8, lane 4) and iii) biotin-labelled HA (Fig. 8, lane 6). Bacterial lysates containing GST but not the RHAMM cDNA plasmid did not react with these reagents (Fig. 8, lanes 1,3,5). These results confirmed the identity of the fusion protein as RHAMM and showed that it bound to biotin-labelled HA as previously reported (53). The ability of RHAMM fusion protein to bind to HA was also confirmed by its ability to bind to HA-Sepharose (data not shown).

### Truncation of RHAMM cDNA

The RHAMM cDNA was cloned into the pGEX-2T plasmid (62,22) as described above. This plasmid contains cloning sites that are followed by three stop codons at three different reading frames (62,22). The recombinant plasmid was digested with restriction endonucleases (a) AatII, (b) BglII + EcoRI, (c) Ncol + EcoRI and (d) SacI + EcoRI. Digests were blunt ended with the Klenow Fragment or, for the EcoRI-SacI fragment, S1 nuclease (Fig. 9). Plasmids containing each truncated RHAMM cDNA respectively were recovered by agarose gel electrophoresis, self-ligated and then transformed into HB101 cells as above. Positive clones were selected by hybridization with RHAMM cDNA and confirmed to contain truncated RHAMM cDNAs by restriction endonuclease digestion and agarose gel electrophoresis to quantitate insert size.

An additional deletion of RHAMM cDNA was achieved using the restriction endonucleases EcoRI and HphI. Plasmid pGEX-2T containing the complete RHAMM cDNA was digested with EcoRI and then blunt ended with the Klenow Fragment. DNA was recovered by ethanol precipitation, suspended in TE buffer, pH 8.0, digested with BglII and purified by agarose gel electrophoresis. The blunt ended EcoRI-BglII fragment (5.66 kb), was purified with a Prep-A-Gene Kit. Conversely, the complete RHAMM cDNA, obtained from the recombinant plasmid, was digested with HphI, blunt ended with S1 nuclease and precipitated with ethanol. DNA was resuspended in TE buffer, pH 8.0, digested with BGlII and purified by agarose gel electrophoresis. The blunt-ended HphI-BglII fragment (477 bp) was isolated, purified with a Prep-A-Gene Kit, ligated to the blunt-ended EcoRI-5 BglII fragment and transformed into HB101. Eight colonies that hybridized with RHAMM cDNA were picked to prepare DNA. DNA samples were digested with BglII and Tth3I to confirm that the correct insert (0.41kb, 0.71kb, 1.02kb and 1.3kb) was present.

The truncated RHAMM cDNAs used in these experiments are shown in Figure 9.

The truncated RHAMM cDNA plasmids were transformed into HB101 and expressed as fusion proteins of 43, 55, 67 and 83 kDa and were detected using anti-GST antibodies (Fig. 10A). These proteins corresponded to the size of protein predicted from the truncations shown in Fig. 9.

Truncated fusion proteins were tested fcr their ability to bind to biotin-labelled HA (Fig. 10B). The complete fusion protein bound HA (lane 6) as did the SacI truncation (lane 5), but the fusion proteins expressed from the NcoI (lane 2), BGlII (lane 3) and AatII (lane 4) truncated RHAMM cDNAs did not stain with biotin-labelled HA. These results indicated that the NcoI-SacI fragment containing a 95 amino acid sequence near the carboxy terminus was critical for binding to HA.

### Effect of salt concentration

It has previously been postulated that positive charge clusters are important in binding glycosaminoglycans, particularly heparin (854-60). To test whether or not charge is important in the binding of HA to RHAMM, the effect of increasing salt concentration on binding of biotin-labelled HA to expressed RHAMM fusion protein was determined.

GSTRHAMM fusion protein was separated on SDS-PAGE and transblotted onto nitrocellulose membrane. Proteins were stained with biotin-labelled HA in the presence of increasing NaCl (150-2000 mM). Results are shown in Figure 11.

As has been determined for heparin-protein interactions (58), the binding of the fusion protein product to biotin-labelled HA was reduced to 19% of the control binding activity when the NaCl concentration was increased from 150 MM to 2,000 mM. These results indicated a role for ionic interactions in HA:RHAMM binding.

### Immunoblots and Transblots using Biotinylated HA

The method described originally by Towbin (63) was used for preparing immunoblots with the following minor modifications. Proteins were separated on 10% SDS-PAGE and transblotted onto nitrocellulose membranes in a buffer containing 25 mM Tris, 192 mM glycine and 20% methanol, pH 8.3. Additional protein binding sites on the membrane were blocked in 10 mM Tris-Cl, pH 8.0, containing 150 mM NaCl (TBS), 0.05% Tween 20 (TBST) and 5% skim milk powder (TBSTS) for 1 h. The blocked membrane was incubated with primary antibody diluted in TBSTS for 1 h at room temperature or overnight at 4°C. The membrane was washed with TBST for 4 x 10 min, and then incubated with the appropriate secondary antibody conjugate diluted in TBST. After washing as above, the membrane was rinsed with TBS. Antibody binding was visualized with a chemiluminescence method (ECL) (64,65) used according to the manufacturer's, instructions (Amersham).

The labelling of hyaluronan with biotin was a modification of the procedure for labelling of antibodies with biotin (66). This method incorporates label onto free amino groups which are present in small amounts within the hyaluronan molecule (67). HA, at an average MW_{E} 500,000 daltoris, was dissolved in 0.1 M sodium borate, pH 8.8, (1.5 µg/µl), iriculated with N-hydroxysuccinimide biotin dissolved in DMSO (7.5 µg/µl) at a ratio of 50:1 (HA:Biotin v/v) at room temperature for 4 h and then incubated with 15 µl of 1 M ammonium chloride for 10 min. The solution was dialysized against PBS, pH 7.2 and then PBS containing 30% glycerol. The linkage of biotin to HA was stable for several months at 4°C and preparation and properties of this compound are described in detail elsewhere (68). It is believed to link to HA via the few amino groups present in this glycosaminoglycan (67). Staining of cell lysates with biotinylated HA was carried out as described above except that labelled HA was substituted for primary antibody and diluted in TBST.

### Identification of two HA binding motifs

Two sequences encoded in the NcoI-SacI fragment (nucleotide ¹²⁰¹⁻¹²³³ and nucleotide ¹²⁶⁷⁻¹²⁹⁶) that was shown to bind to HA in the above section were noted to contain clusters of positive charges. To determine whether these motifs were involved in the binding of HA to RHAMM, three approaches were used. In the first approach, the region was deleted and then the resulting expressed truncated fusion protein was tested for its ability to bind to HA (Fig. 12A&B). In the second approach, peptides were synthesized that mimicked the two regions containing positive charge clusters and then tested for their ability to HA-Separose and to compete with RHAMM for binding biotin-labelled HA in a transblot assay (Fig. 13A&B). In the third approach, oligonucleotides encoding the putative binding domains were genetically engineered, using PCR, to the 0.71 Kb cDNA (non-HA binding) encoding the amino terminus of RHAMM. The altered fusion protein products were then tested for their ability to bind to HA (Fig. 14A&B).

In the first approach, a deletion of HphI-SacI cDNA fragment that contained the clusters of basic amino acids was made as described above. A correct religation of the deleted cDNA was determined by restriction mapping. The DNA sample having a BglII site and a shorter fragment after BglII-Tth3I digestion implied that a deletion of HphI-EcoRI fragment had occurred and that a correct religation ensued (Fig. 12A). The colonies containing this correct religated plasmid were used to make GST-fusion protein as described above. The GST-RHAMM fusion protein was electrophoresed as a 74 kDa protein, which was the size predicted for this deletion. In a transblot procedure using biotin-labelled HA, the 74 kDa deleted protein had lost its ability to bind to HA (Fig. 12B, lane 3). This result defined the critical HA binding site of RHAMM as the region between aa 400-434 occurring within the HphI and SacI sites marked in Fig. 9.

The fusion protein from RHAMM cDNA truncated with SacI and EcoRI showed binding to HA (Figure 12B, lane 2).

To further assess the role of the deleted region in binding to HA, peptides corresponding to the two domains containing clusters of basic amino acid residues, K⁴⁰¹Q K I K H V V K L K⁴¹¹, K⁴²³L R S Q L V K R K⁴³², were synthesized and used to compete with binding of biotinylated HA to the complete GST-RHAMM fusion protein.

Transblotted RHAMM fusion protein was stained with a 1:10,000 dilution of biotin-labelled HA that had been pre-incubated for 1 h with 3 mg/ml BSA (Figure 13A, lane 1) , 3 mg/ml peptide^{aa401-411} (lane 2) or 3 mg/ml peptide^{aa423-432} (lane 3). Results showed that peptide^{aa401-411} and peptide^{aa423-} ⁴³² blocked the binding of biotin-labelled HA to expressed GST-RHAMM fusion protein.

To directly demonstrate the ability of peptide^{aa401-411} and peptide^{aa423-432} to bind to HA, peptides were chromatographed on HA-Separose.

HA-Sepharose affinity gel was prepared according to the manufacturer's instruction. RHAMM peptides (peptide^{aa401-411}; peptide^{aa423-432}; randomized peptide^{aa401-411} (L K Q K V K K H I V); randomized peptide^{aa423-432} (Q S K R L K K R V L) ; peptide^{aa125-145} and peptide^{aa269-288}, (53) were applied to HA-Sepharose. Unbound peptides were removed by washing the gel with PBS containing 0.15 M NaCl. The amounts of peptides applied and the unbound peptide removed from the gel were determined by measuring their O.D. value. Results are shown in Figure 13B.

Several controls were included to evaluate the role of non-specific binding. BSA, as well as a peptide^{aa125-145} mimicking a repeated sequence encoded in the RHAMM amino terminus and a peptide^{aa269-288} mimicking a hydrophilic region encoded in the center of the RHAMM cDNA (53) were all tested for their ability to bind to HA-Sepharose. As well, to investigate the importance of the spacing of basic amino acids, two random peptides that contained the same amino acid residues as peptide^{aa401-411} and peptide^{aa423-432} were also tested for their ability to bind to HA sepharose. These results showed that only peptide^{aa401-411} and peptide^{aa423-432} efficiently bound to HA (Fig. 13B).

DNA sequences encoding peptide^{aa401-411} and peptide^{aa23-432} were each linked to the BgIII truncated RHAMM cDNA which encodes the 27.5 kDa polypeptide of the N-terminus of RHAMM.

### Construction of Recombinant RHAMM Containing Oligonucleotides Encoding HA-Binding Peptides

PCR was used to incorporate the HA binding regions (peptide^{aa401-411} and peptide^{aa423-432} respectively) into a cDNA encoding the N-terminus of RHAMM that was prepared as a 0.71 Kb fragment (aa 1-238, see above and Fig 9). The fusion protein product of this fragment did not have the ability to bind HA (Fig. 14). The procedure was carried out by making two PCR primers (5'TAG AAT GAA TTC TTT CAA TTT CAC AAC ATG TTT GAT TTT TTG TTT AAG ATC TTC TAT TTC and 5'TAG AAT GAA TTC TTT CCT TTT AAC AAG CTG AGA TCG CAG TTT AAG ATC TTC TAT TTC) which contained both a region mimicking the oligonucleotides encoding either peptide^{aa401-} ⁴¹¹ and peptide^{aa423-432} (creating an EcoRI site at the end of each primer) and a region mimicking 18 bp of the 3' end cDNA of the 0.71 Kb insert. Recombinant cDNA was obtained with a PCR reaction by using either of these two primers together with a primer that mimicked the 5' end of the RHAMM cDNA (nucleotide 1-22) (creating a BamHI site) with the same conditions described in the construction of RHAMM cDNA. Both PCR products were digested with EcoRI and BamHI and purified in 1% agarose gel electrophoresis. Recombinant cDNAs were then inserted into pGEX-2T opened with BamHI and EcoRI and transformed into HB101 as above. The correct insertion of the recombinant cDNAS was confirmed by restriction endonuclease digestion of the selected clones and by sizing of the insert with agarose gel electrophoresis.

These recombinant sequences were then expressed as recombinant proteins (Fig 14A). The HA binding ability of the resulting recombinant proteins was tested in transblot assays.

Bacterial cell lysates containing the GST-RHAMM fusion proteins were fractionated on SDS-PAGE, trans blotted onto nitrocellulose membrane and visualized with either polyclonal antibody to peptide^{aa125-145} (lane 1-3) or biotinlabelled HA (lane 4-6). The GST fusion non-recombinant polypeptide^{aa1-238} was used as a control (lane 1 and lane 4). The linkage of either peptide^{aa401-411} (lane 2 and lane 5) or peptide^{aa423-432} (lane 3 and lane 6) to the N-terminal RHAMM polypeptide^{aa1-238} created HA binding domains (lane 5-6) although their antibody binding properties remained the same (lane 2 -3).

Truncated RHAMM fusion proteins containing either the peptide^{aa401-411} (lane 5) or the peptide^{aa423-432} (lane 6) bound efficiently to HA (Fig 14B). In contrast, and as noted in Fig 10B, the fusion protein containing only the amino terminus of RHAMM did not bind to HA (Fig. 14B, lane 4). These results indicated that each genetically engineered alteration of the amino terminus encoded in RHAMM cDNA created an HA binding site, indicating that both peptide^{aa401-411} and peptide^{aa423-432} comprise the HA binding domains of RHAMM.

### Blocking of HA Binding with RHAMM Peptides

GST-RHAMM fusion protein was electrophoresed on 10% SDS-PAGE and transblotted onto a nitrocellulose membrane. The membrane was cut into 3 strips and each was stained with 1:3000 diluted biotin-labelled HA in the presence of 3 mg/ml BSA, 3 mg/ml peptide^{aa401-411} and 3 mg/ml peptide^{aa423-432}, respectively. The other steps were the same as described in immunoblots.

### Inhibition of HA-stimulated locomotion by RHAMM peptides

Peptides K⁴⁰¹ Q K I K H V V K L K⁴¹¹ and K⁴²³ L R S Q L V K R L⁴³² were synthesised and their effect on HA-stimulated locomotion in ras-transformed fibroblast cells was examined.

Peptides (1 ng to 500 µg/ml) were added to cells and their effect on cell locomotion was monitored with an imager analysis system as described (53).

The results are shown in Figure 15.

### Hyaluronan-Sepharose Affinity Chromatography

Hyaluronan (Sigma) was coupled to AH-Sepharose 4B (Pharmacia) according to the manufacturer's instructions, resulting in an HA-Sepharose gel. Peptides, ordered from Coast Scientific (San Diego) were HPLC purified. These and randomized peptides, whose sequence was generated by computer, were applied to HA-Sepharose in the presence of 0.15 M NaCl in PBS buffer. Unbound peptides were removed by extensive washing with the same buffer. The amounts of peptides applied and that removed were determined by measuring optical density (O.D.) at 214 nm.

### EXAMPLE 3

### Materials

Biotin-labelled HA was prepared by dissolving the polymer (1.5 µg/µl) in 0.1 M sodium borate, pH 8.8, incubating with N-Hydroxysuccinimide biotin dissolved in DMSO (7.5 µg/µl) at a ratio of 50:1 (HA:Biotin) at room temperature for 4 h and then incubating the reacted products with 15 µl of 1 M ammonium chloride for 10 min. The solution was dialysed against PBS, pH 7.2 and then PBS containing 20% glycerol. The biotin-labelled HA could be stored at 4°C for several months. Staining of cell lysates with biotinylated HA was carried out as described below (transblot assay) except that labelled HA was substituted for primary antibody and diluted in TBST.

Peptides were synthesized by Dr. David Litchfield, Manitoba Institute of Cell Biology. The purity of synthetic peptides were determined on HPLC at optical densities (OD) 280 and 214. Oligonucleotides were synthesized in the Dept. of Physiology, University of Manitoba. Oligonucleotides longer than 30 bases were purified on HPLC. AH-Sepharose power was obtained from Pharmacia.

### Polymerase Chain Reaction (PCR)

PCR reactions were performed as previously described (61) using a standard (P)[?] kit (Amersham) . The reaction mixture (100 µl) normally contained 1.25 nM MgCl₂, 75 µM dNTP, 1 µg of each primers (in excess), 5 ng template DNA and 1 unit of Taq DNA polymerase. All reactions were carried out at 94°C (30 sec), 54°C (30 sec) and 72°C (90 sec) for 25 cycles.

### DNA Purification and Ligation

DNA products from the PCR reaction mixtures were purified using Prep-A-Gene DNA Purification kit. The reaction mixture was brought to 1 ml by adding 25 µl Prep-A-Gene Matrix and 875 µl binding buffer. After washing twice in binding buffer and 3 times in wash buffer, the DNA was eluted in 100 µ H₂O. The DNA was ligated to plasmids as described (94) using a DNA ligase (Boehringer Mannheim).

### Transformation, DNA Preparation and Digestion with Restriction Endonucleases

Competent bacteria were prepared as described (61) with some modifications. Bacteria (E. coli HB101 or XL1-Blue), stored in glycerol, were grown in 1 ml LB/amp medium at 37°C overnight. The resultant culture was brought to 10 ml with LB/amp medium and Mg₂SO₄ was added to a concentration of 10 mM. The culture was incubated at 37°C for 2 h with constant agitation. The cells were chilled on ice for 20 min. pelleted at 1,000 xg for 5 min at 4°C, resuspended in 3 ml 50 mM CaCl₂, re-chilled on ice for 20 min, pelleted at 1,000 xg for 5 min at 4°C, resuspended in 500 µl 50 mM CaCl₂ and put on ice for transformation. Transformation was carried out exactly as previously described (61). The transformed mixtures were spread on 3 LB/amp agar plates for HB101 or 3 LB/amp agar plates containing isopropylthio-β-D-galactoside (4 mg/plate) and x-gal (0.2 mM/plate) for XL1-Blue. The plates were incubated at 37°C overnight. Twelve white colonies of XL1-Blue or twelve randomly picked colonies of HB101 were streaked on LB/amp agar plate for incubation at 37°C overnight and inoculated in Eppendorf tubes containing 1 ml LB/amp medium for incubation at 37°C overnight with shaking at 280 rpm.

DNA's were prepared from the above 1 ml culture as described (61) with modifications. The cells were sedimented and resuspended in 100 µl solution I. Cells from each tube were lysated with µg lysozyme dissolved in 10 µl solution I and with 200 µl 0.2 N NaOH containing 1% SDS at room temperature for 10 min. 150 µl solution III was added to each lysate and put on ice for 15 min. Genomic DNA and bacterial debris were sedimented with 10,000 xg for 15 min. Plasmid DNA in the supernatant was precipitated with 0.6 volume of isopropyl alcohol at room temperature for 10 min and then by centrifugation at 10,000 xg for 15 min. The DNA was resuspended in 30 µl TE buffer, pH 8.0, and purified with Prep-A-Gene kit using 5 µl Prep-A-Gene Matrix washed twice with binding buffer and twice with was buffer. The purified DNA was eluted in 50 µl H₂).

DNA samples were doubly digested with two restriction endonucleases (i.e. BamHI+Sacl, BamHI+EcoRI, BamHI+ClaI or ClaI+EcoRI) and electrophoresed on an agarose gel to select those clones containing correct inserts. For experiments using Bluescript, the correct insert was recovered for further use by Prep-A-Gene DNA Purification kit. For experiments using pGEX-2T (62,22), the clones containing the correct insert were used for preparation of fusion proteins.

### Site-directed mutagenesis of HA binding domain (peptide^{aa423-432)} in RHAMM.

Four cDNAs containing site-directed mutations in each cDNA were generated in four PCR reactions. The template DNA was the part of RHAMM cDNA (nucleotide 1-714) containing the oligonucleotide encoding peptide^{aa423-432} of RHAMM. The primers in each reaction were the one complimentary to translation initiation region and one of the 4 primers containing mutated nucleotides as in Figure 17. PCR products from the 4 primers containing mutated nucleotides were doubly digested with BamHI + EcoRI, ligated into pGEX-2T and transformed into HB101.
Selected clones were confirmed to contain the inserts by double digestion with BamHI + EcoRI and electrophoresis on agarose gel, as shown in Figure 18A. Fusion proteins were prepared from the selected clones, proceeded to Western blot and stained with anti-RHAMM antibody, (Figure 18B) and with biotin-labelled HA (Figure 18C).

### Genetic manipulation of link protein peptide^{aa316-325}

Peptide^{aa316-325} of link protein (R Y P I S R P R K R) has been shown to contain an HA binding domain (8).

An oligonucleotide encoding peptide^{aa316-325} of link protein was aligned to a part of RHAMM cDNA encoding a polypeptide that did not bind to HA, polypeptide^{aa1-238}, using PCR techniques, as shown in Figure 19A.

This was carried out by having a primer that was complimentary to nucleotide 358-375 of RHAMM and nucleotide 946-975 of link protein creating an EcoRI site. Both of them were in the same reading frame. The recombinant cDNA as generated using this primer and another primer complementary to nucleotide 1-22 of RHAMM creating a BamHI site at nucleotide 1. The PCR product was then doubly digested with BamHI + EcoRI, ligated into pGEX-2T, transformed into HB101 and confirmed to be present in the selected clones. The strategy is illustrated in Figure 19A. Western Blot assays of the fusion proteins obtained from these clones were performed and the results are shown in Figure 19B.

Fusion proteins were prepared from the original clone containing RHAMM peptide^{aa1-238} only (lane 1 and 4) and the clone additionally containing peptide^{aa316-325} of link protein (lanes 2 and 5). HB101 lysate was used as a control (lanes 3 and 6). The fusion proteins on Western blot assay were stained with anti-RHAMM antibody (lanes 1-3) and biotin-labelled HA (lanes 4-6). The results showed that only fusion protein containing peptide^{aa316-325} of link protein had the ability to bind to biotin-labelled HA (lane 5).

### Site-Directed mutagenesis of HA binding domain (peptide^{aa-401-411}) in RHAMM

The strategy of the site-directed mutagenesis is shown in Figure 17.

Two primers within peptide^{aa401-411} were synthesized which generated 3 site mutations as required and created a ClaI site in each primer (Figure 18). Combined with the other two primers which were used to amplify the complete RHAMM cDNA reading frame, the RHAMM cDNA were generated into two pieces overlapped with a ClaI site in two PCR reactions. The two ClaI sites were in the same reading frame of the cDNA. After PCR amplification, Part I (Figure. 18A, lane 1) was doubly digested with BamHI + ClaI and ligated into BamHI-ClaI opened Bluescript. Part II (Fig. 18A, lane 2) was doubly digested with ClaI + EcoRi and ligated into ClaI-EcoRI opened Bluescript. The ligation mixtures were transformed into E. coli XL1-Blue. DNAs were prepared from white colonies and doubly digested by BamHI + ClaI or ClaI + EcoRI to select the clones containing appropriate inserts (Fig. 18A, lane 3 and 4). The inserts were recovered with Prep-A-Gene kit, doubly ligated into BamHI-EcoRI opened Bluescript and proceeded as above to select a proper insert containing Part I and Part II (Fig. 18A, lane 5).

The insert was recovered and ligated into BamHI-EcoRi opened pGEX-2T and transformed into HB101. The clone containing expected insert identified by restriction endonuclease digestion (Fig. 18A, lane 6) was selected and used to prepare fusion protein. Fusion proteins (Fig. 18B, lanes 1 and 3) were subjected to Western blot assay stained with antibody to RHAMM (Fig. 18B, lanes 1-2) and biotin-labelled HA (Fig. 18B, lanes 3-4). Non-mutated RHAMM fusion protein was used as control (Fig. 18B, lanes 2 and 4). The results (Fig. 18B, lane 4) showed that after the alteration of Lys⁴⁰⁵, Lys⁴¹¹ and Lys⁴²³ to Glu⁴⁰⁵, Glu⁴¹¹ and Glu⁴²³, the HA binding ability of RHAMM was severely compromised.

### Site-directed mutation of RHAMM

21A. Part I and Part II were amplified in PCR reactions (lane 2 and 3).

21B. The PCR products were doubly digested with BamHI + ClaI (for Part I) and ClaI + EcoRI (for Part II), ligated to properly opened Bluescript and transformed into E. coli XLi-Blue. DNAs were prepared from white colonies and confirmed to contain appropriate inserts by digestion with proper restriction endonucleases (lane 2 and 3). The two DNAs were doubly ligated into a Bluescript and confirmed to be present in the selected clones (lane 4). This insert, containing the mutated complete RHAMM cDNA was recovered and ligated into pGEX-2T and confirmed to present in that plasmid by digestion with BamHI + EcoRI (lane 5).

### Peptide^{aa401-441} and peptide^{aa423-432} are the only HA binding domains in RHAMM

These two peptide sequences of RHAMM are close together and followed by an unique SacI site within the RHAMM cDNA reading frame (Fig. 1). RHAMM cDNA from nucleotide 1-1230 was amplified in PCR. In this way, HA binding domain peptide^{aa423-432} was completely deleted while the other HA binding domain peptide^{aa401-411} was partially deleted. The basic amino acids in the remaining domain were mutated (Fig. 1) to completely abolish its HA binding ability. The PCR product was inserted into pGEX-2T containing SalI-EcoRI fragment and transformed into E. coli HB101. DNA samples prepared from randomly picked clones were confirmed to contain correct inserts.

The colony containing a correct BamHI-SacI insert was used to made fusion proteins. Undeleted RHAMM fusion protein and HB101 lysate were used as controls. The fusion proteins were subjected to Western blot assays stained with antibody to RHAMM and biotin-labelled HA (data not shown). The results showed that the deleted fusion protein completely lost its ability to bind to HA indicating that these two HA binding domains were the only HA binding domains in RHAMM.

The present invention is not limited to the features of the embodiments described herein, but includes all variations and modifications within the scope of the claims.

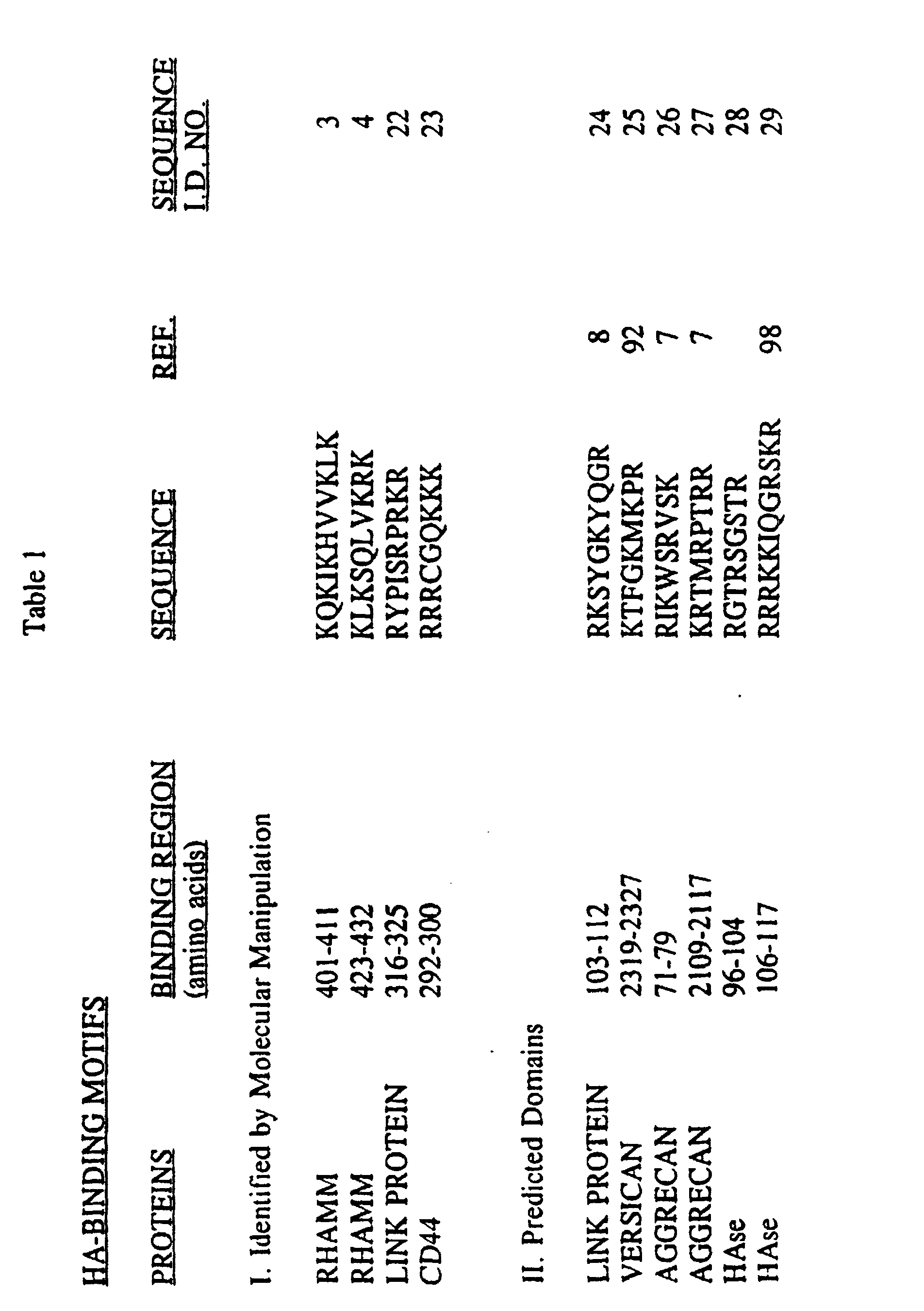

### REFERENCES

1. Barondes, S.H. (1988) Bifunctional properties of lectins: lectins redefined. Trends Biochem. Sci. 13, 480-482.
2. Boudreaux, N., Turley, E.A., and Rabinovitch, M. (1991) Fibronectin, hyaluronan and a hyaluronan binding protein contribute to increased ductus ateriosus smooth muscle cell migration. Devel. Biol-143
3. Brown, T., Bouchard, T., St. John, T., Wagner, E. and Carter, G. (1991) Human keratinocytes express a new CD44 core protein (CD44E) as a heparin-sulfate intrinsic membrane proteoglycan with additional Exons. J. Cell Biol. 113: 207-221.
4. Chen, W.Y., Grant, M.E., Schor, A.M. and Schor, S.L. (1989) Differences between adult and fetal fibrobrasts in the regulation of hyaluronate synthesis: correlation with migrating activity. J. Cell. Sci. 94, 577-584.
5. Chou, P.Y. and Fasman, G.D. (1978) Pediction of the secondary structures of proteins from their amino acid sequence. Adv. Enzymol. 47, 45-148.
6. Choy, B.K., McClarty, G.A. and Wright, J.A. (1989) Transient elevation of ribonucleotide reductase activity, M2 mRNA and M-2 protein in BALB/C 3T3 fibroblasts in the presence of 12-0-teracycanoly-phorbol-13-acetate. B.B.R.C. 162, 1417-1424.
7. Doege, K., Sasaki, M., Horigan, E., Hassell, J.R. and Yamada, Y. (1987) Complete primary structure of the rat cartilage proteoglycan core protein deduced from cDNA clones. J. Biol. Chem. 262, 17757-17767.
8. Goetinck, P.F., Stirpe, N.S., Tsonis, P.A. and Carlome, D. (1987) The tandomly repeated sequences of cartilage link protein contain the sites for interaction with hyaluronic acid. J. Cell Biol. 105, 2403-2408.
9. Holland, E.C., Leung, J.D. and Drickamer, K. (1984) Rat liver asialoglycoprotein receptor lacks a cleavable NH₂-terminal signal sequence. Proc. Natl. Acad. Sci. USA 81, 7338-7342.
10. Ikuta, K., Takami, M., Kim, C.W., Honjo, T., Miyoshi, T., Tagaya, Y., Kawabe, T. and Yodoi, J. (1987) Human lymphocyte Fc receptor for IgE: sequence homology of its cloned cDNA with animal lectins. Proc. Nail. Acad. Sci. USA 84, 819-823.
11. Krusius, T., Gehlsen, K.R. and Ruoslatti, E. (1987) A fibroblast chrondroitin sulfate proteoglycan core protein contains lectin-like and growth factor-like sequences. J. Biol. Chem. 262, 13120-13125.
12. Kuhn, L.A., Griffin, J.H., Fisher, C.C., Greengard, J.S., Bouman, B.N, Espana, F., and Tainer, J.A. (1990) Elucidating the structural chemistry of glycosaminoglycan recognition by protein C inhibitor. Proc. Natl. Acad. Sci., USA. 87, 8506-8510.
13. Longaker, M.T., Chiu, E.S., Harrison, M.R., Crombleholne, T.M., Langer, J.C., Duncan, B.W., Adzick, N.S., Verrier, E.D., and Stern, R. (1989) Studies in fetal wound healing IV. Hyaluronic acid stimulating activity distinguishes fetal wound fluid from adult wound fluid. Ann. Surg. 210, 667-672.
14. Liotta, L.A., (1986) Tumor cell autocrine motility factor. Proc. Natl. Acad. Sci. USA. 83:3302-3306.
15. McClelland, A., Kuhn, L.C. and Ruddle, F.H. (1984) The human transferrin receptor gene: genomic organization and the complete primary structure of the receptor deduced from cDNA sequence. Cell 39, 267-274.
16. Neame, P.J., Christner, J.E. and Baker, J.R. (1986) The primary structure of link protein from rat chondrosarcoma proteoglycan aggregate. J. Biol. Chem. 261, 3519-3535.
17. Partin, A.W., Isaacs, J.T., Trieger, B. and Coffey, D.S. (1988) Early cell motility changes associated with an increase in metastatic ability in rat prostatic cancer cells transfected with the Harvey ras oncogene. Cancer Res. 48, 6050-6053.
18. Rao, C.N., Castronova, V., Schmitt, M.C., Wiwer, U.M., Claysmith, A.P., Liotta, L.A. and Sobel, M.E. (1989) Evidence for a precursor of the high affinity metastasis-associated murine laminin receptor. Biochem. 28, 7476-7486.
19. Ruoslahti, E and Yamaguchi, Y. (1991) Proteoglycans as modulators of growth factor activity, cell, 64: 867-869.
20. Schor, S.L., Schor, A.M., Grey, A.M., Chen, J., Rushton, C., Grant, N.E. and Eins, 1. (1989) Mechanisms of action of the migration stimulating factor produced by fetal and cancer patient fibroblasts: effect on hyaluronic acid synthesis. In Vitro Cell Devel. Biol. 25, 737-745.
21. Shanley, D.J., Cossu, G., Boettiger, D., Focht, R.J., Holtzer, H. and Pacifica, M. (1983) Transformation by Rous sarcoma virus induces similar patterns of glycosaminoglycan synthesis in chick embryo skin fibroblasts and vertebral chondroblasts. J. Biol. Chem. 258, 810-816.
22. Smith, D.B. and Johnson, K.S. (1988). Single-step purification of polypeptides expressed in E. coli asffusions with glutathione S-transferase. Gene 67:31-40.
23. Stamenkovic, I., Amiot, M., Pesando, J.M. and Seed, B. (1989). A lymphocyte molecule implicated in lymph node homing is a member of the cartilage link protein family. Cell 56, 1057-1062.
24. Stamenkovic, I., Aruffo, A., Amist, M. and Seed, B. (1991). The hematopoietic and epithelial forms of CD44 are distinct polypeptides with different adhesion potentials for hyaluronan-bearing. Cells Embo J. 10: 343-348.
25. Toole, B.P., Munaim, S.F., Welles, S. and Knudson, C.B. (1989) Hyaluronate-cell interactions and growth factor regulation of hyaluronate synthesis during embryo development. In Biology of Hyaluronan eds. Evered, D. and Whelan J. (John Wiley and Sems, Chichester), vol. 143, pp 146-159.
26. Triable, W.S., Johnson, P.W., Hozumi, N. and Roder, J.C. (1986) Inducible cellular transformation by a metallothionein-ras hybrid oncogene leads to natural killer cell susceptibility. Nature 321, 782-785.
27. Turley, E.A. (1984) Proteoglycans and cell adhesion: their putative role during tumorigenesis. Cancer Met. Rev. 3, 325-339.
28. Turley, E.A. and Torrance, J. (1985) Localization of hyaluronate and hyaluronate binding protein on motile and non-motile fibroblasts. Exp. Cell. Res. 161, 17-28.
29. Turley, E.A., Moore, D., and Hayden, L.J. (1987) Characterization of hyaluronate binding proteins isolated from 3T3 and murine sarcoma virus transformed 3T3 cells. Biochemistry 26, 2997-3005.
30. Turley, E.A. (1989) Hyaluronic acid stimulates protein kinase activity in intact cells and in an isolated protein complex. J. Biol- Chem. 264, 8951-8955.
31. Turley, E.A. (1989) The role of a cell associated hyaluronan-binding protein in fibroblast behaviour. In Biology of Hyaluronan eds. Evered, D. and Whelan, J. (John Wiley and Sons, Chichester), vol-143, pp 121-137.
32. Turley, E.A. and Auersperg, N. (1989) A hyaluronate binding protein transiently codistributes with p21, k-ras in cultured cell lines. J. Exp. Cell Res., 181, 340-348.
33. Turley, E.A., Brassel, P. and Moore, D. (1990) A Hyaluronan binding protein shows a partial and temporally regulated codistribution with actin on locomoting chick heart fibroblasts. Exp. Cell. Res. 187, 243-249.
34. Turley, E.A., Austen, L., Vandeligt, K. and Clary, C. (1991) Hyaluronan and a cell-associated Hyaluronan binding protein regulate the locomotion of ras-transformed cells. J. Cell Biol. 112, 1041-1047.
35. Turley, E.A., Hoare, K. and Cripps, V. (1991), in press.
36. Yow, H.K., Wong, J.M., Chen, H.S., Lee, C.G., Steele, G.D.J. and Chen, L.B. (1988) Increased mRNA expression of a laminin-binding proteins in human colon carcinoma: complete sequence of full length cDNA encoding the protein. Proc. Natl. Acad. Sci. USA 85, 6394-6398.
37. Zakut-Houri, R., Oren, M., Brenz, B., Lavie, V., Hazun, S. and Gwol, D. (1983) A single gene and a pseudogene for the cellular tumor antigen p 53. Nature 306, 594-597.
38. Kivst, T.N. and Finnegan, C.V. (1970) J. Exp. Zool. 175, 241-246.
39. Toole, B.P. (1982) Conn. Tiss. Res. 10, 93-100.
40. Iozzo, R.V. (1985) Lab. Invest. 53, 373-396.
41. Markwald, R.R., Fitzharris, T.P., Bank, H. and Bernanke, D.H. (1978) Devel. Biol. 62, 292-316.
42. McGlay, D.R. and Ettensohn, C.A. (1987) Ann. Rev. Cell Biol. 3, 319-345.
43. McCarthy, M.T. and Toole, B.P. (1989) J. Cell Physiol. 141, 191-202.
44. McGary, C.T., Raja, R.H. and Weigel, P.H. (1989) Biochelfi. J. 25v, 875-884.
45. Bowen, B.R., Nguyen, T. and Lasky, LA. (1989) J. Cell Biol. 109, 421-427.
46. Fosang, A.J. and Hardingham, T. (1989) Biochein. J. 269, 801-809.
47. Deak, F., Kiss, I., Sparks, K.J., Argraves, W.S., Hampikian, G. and Goetinck, P.F. (1986) Proc. Natl. Acad. Sci. U.S.A. 83, 3766-3770.
48. Doege, K., Hassell, J.R., Caterson, B. and Yamada, Y. (1986) Proc. Natl. Acad. Sci. U.S.A. 83, 3761-3765.
49. Kiss, I., Deak, F., Mestric, S., Delius, ti., Soos, J., Dckaiicj, K., Argraves, W.S., Sparks, K.J. and Goetinck, P.F. (1987) Ilroc. Natt. Acad. Sci. U.S.A. 84, 6399-6403.
50. Goldstein, L.A., Zhou, D.F.H., Picker, L.J., Mi-nty, C.N., Bargatze, R.F., Ding, J.F. and Butcher, E.C. (1989) Cell, 56, 1063-1072.
51. Lee, T.H., Wisniewski, H.G. and Viicek, J. (1992) J. Cell Biol. 116, 595-557.
52. LeBaron, R.G., Zimmermann, D.R. and Ruoslahti, E.(1992) J. Biol. Chem., 267, 10003-10010.
53. Hardwick, C., Hoare, K., Owens, R., Hohn, H.P., Hook, M., Moore, D., Cripps, V., Austen, L., Nance, D.M. and Turley, E.A. (1992) J. Cell Biol. 117, 1343-1350.
54. Gandrille, S., Aiach, M., Lane, D.A., Videaud, D., Molho-Sabatier, P., Caso, R., de Moerloose, P., Fiessinger, J.N. and Clauser, E. (1990) J. Biol. Chem. 265, 18997-19001.
55. Kost, C., Stuber, W., Ehrlich, H.J., Pannekoek, H. anci Preissner, K.T. (1992) J. Biol. Chem. 267, 12098-12105.
56. Pratt, C.W. and Church, F.C. (1992) J. Biol. Chem. 267, 8789-8794.
57. Kost, C., Stuber, W., Ehrlich, J.J., Pannekoek, H. and Preissner, K.T. (1992) J. Biol. Chem. 267, 12098-12105.
58. Ehrlich, H.J., Gebbink, R.K., Keijer, J. and Pannekock, H. (1992) J. Biol. Chem. 267, 11606-11611.
59. Sobel, M., Soler, D.F., Kermode, J.C. and Harris, R.B. (1992) J. Biol. Chem. 267, 8857-8862.
60. Pratt, C.W., Whipna, H.C. and Church, F.C. (1992) J. Biol. Chem. 267, 8795-8801.
61. Sambrook, J., Fritsch, E.F. and Maniatis, T. (1989) Molecular cloning: a laboratory manual. Cold Spring Harbor Laboratory, Cold Spring Harbor, New York.
62. Eaton, D., Rodriguez, H. and Vehar, G.A. (1986) Biochemistry 25, 505-512.
63. Towbin, H., Staehelin, T. and Gordon, J. (1979) Proc. Natl. Acad. Sci. USA 76, 4350-4354.
64. Isacsson, H. and Watermark, G. (1974) Anal. Chim. Acta. 68, 339-362.
65. Roswell, D.F. and White, E.H. (1978) Methods in Enzymology 57, 409-423 Deluca, M.A. (Ed.) Academic Press, New York.
66. Bayer, E.A. and Wilchek, M. (1980) Methods Biochem. Anal. 26, 1-45.
67. Hardingham, T.E. and Fosang, A.J. (1992) FASEB J. 6, 861-870.
68. Hoare, K., Savani, R.C., Wang, C., Yang, B. and Turley, E.A., in press.
69. Samuel, S. K., Hurta, R. A. R., Spearrran, M. A., Wright, J. A., Turley, E. A: and Greenberg, A.H., in press.
70. Aruffo, A., Stamenkovic, I., Melnick, M., Underhill, C.B. and Seed, B. (1990) Cell 61, 1303-1313.
71. Culty, M., Miyake, K., Kincade, P.W., Silorski, E., Butcher, E.C. and Underhill, C. (1990) J. Cell Biol. 111, 2765-2774.
72. Doege, K.J., Sasaki, M., Kimura, T. and Yamada, Y. (1991) J. Biol. Chem. 266, 894-902.
73. Gunthert, U., Hofmann, M., Rudy, W., Reber, S., Zoller, M. , HauBmann, I., Matzku, S., Wenzel, A., Ponta, H. and Herrlich, P. (1991) Cell 65, 13-24.
74. Hughes, E., Colombatti, A. and August, J. (1983) J. Biol. Chem. 258, 1014-1021.
75. Hyman, R., Lesley, J. and Schulte, R. (1991) Immunogenetics 33, 392-395.
76. Jackson, D.G., Buckley, J. and Bell, J.I. (1992) J. Biol. Chem. 267, 4732-4739.
77. Kimata, K., Hascall, V.C. and Kimura, J.H. (1982) J. Biol. Chem. 257, 3827-3832.
78. Lesley, J., Schulte, R. and Hyman, R. (1990) Exp. Cell Res. 187, 224-233.
79. Lesley, J., He, Q., Miyake, K., Hamann, A., Hyman, R. and Kincade, P.W. (1992) J. Exp. Med. 175, 257-266.
80. Lyon, M. (1986) Biochim. Biophys. Acta. 881, 22-29.
81. McDevitt, C., Marcelino, J. and Tucker, L. (1991) FEBS LETTERS 294, 167-170.
82. Miyake, K., Underhill, C.B., LeBley, J. and Kincade, P.W. (1990) J. Exp. Med. 172, 69-75.
83. Neame, P.J., Perin, J.P., Bonnet, F., Christner, J.E., Jolles, P. and Baker, J.R. (1985) J. Biol. Chem. 260, 12402-12404.
84. Neame, P.J., Christner, J.E. and Baker, J.R. (1987) J. Biol. Chem. 262, 17768-17778.
85. Perides, G., Biviano, F. and Bignami, A. (1991) Biochimica et Biophysica Acta 1075, 248-258.
86. Tenglad, A. (1981) Biochem. J. 199, 297-305.
87. Timmons, T. and Dunbar, B. (1990) Methods in Enzymology 182, 679-688.
88. Trowbridge, I., Lesley, J., Schulte, R., Hyman, R. and Trotter, J. (1982) Immunogenetics 15, 299-312.
89. Underhill, C.B., Thurn, A.L. and Lacy, B.E. (1985) J. Biol. Chem. 260, 8128-8133.
90. Underhill, C.B., Green, S.J., Comoglio, P.M. and Tarone, G. (1987) J. Biol. Chem. 262, 13142-13146.
92. Zimmermann, D.R. and Ruoslahti, E. (1989) The EMBO J. 8, 2975-2981.
93. Klews. L., Turley, E. and Prehm, P. (1993), Biochem. J., 290, 791-795.
95. Butcher, E.C., (1989), Cell, v. 56, pp. 1063-1072.
96. Turley, E.A. et al., (1993), Blood, v. 81, pp. 446-453.
97. Pilarski, L.M. et al., (1993) in press.
98. Hope, et al. (1993), J. Cell. Biochem., Suppl. 17E, p.175, R 2413.

## Claims

1. An isolated DNA molecule comprising a nucleotide sequence encoding a locomotion-associated hyaluronan binding protein wherein the nucleotide sequence is one of the following sequences:
(i) the nucleotide sequence of Figures 23A, 23B and 23C (Sequence ID No. 1);
(ii) nucleotide sequence 147 - 1428 of Figures 23A, 23B and 23C.

2. A DNA molecule according to claim 1, encoding the derived amino acid sequence of Figure 23A, 23B and 23C (Sequence ID No. 2) commencing with the first methionine, or a fragment thereof which retains the ability to bind hyaluronan.

3. A DNA molecule in accordance with claim 1, encoding the derived amino acid sequence of Figures 23A, 23B and 23C commencing with the second methionine, or a fragment thereof which retains the ability to bind hyaluronan.

4. An isolated locomotion-associated hyaluronan binding protein comprising the derived amino acid sequence of Figures 23A, 23B and 23C, or a fragment thereof which retains the ability to bind hyaluronan.

5. A protein in accordance with claim 4 comprising the amino acid sequence of Figures 23A, 23B and 23C commencing with the second methionine, or fragment thereof which retains the ability to bind hyaluronan.

6. A hyaluronan-binding peptide wherein the peptide is one of the following peptides:
(i) 
Lys - Gln - Lys - Ile - Lys - His - Val - Val -
Lys;
(ii) 
Lys - Ile - Lys - His - Val - Val - Lys - Leu -
Lys;
(iii) 
Lys - Leu - Arg - Ser - Gln - Leu - Val - Lys -
Arg;
(iv)

Lys - Leu - Arg - Ser - Gln - Leu - Val - Lys -
Arg - Lys.

7. An isolated DNA molecule encoding a peptide in accordance with claim 6.

8. An antibody specific for a peptide in accordance with claim 6.

9. A pharmaceutical composition comprising an effective amount of one of the following and a pharmaceutically acceptable carrier:
(i) a protein in accordance with any of claims 4 or 5;
(ii) a peptide in accordance with claim 6;
(iii) an antibody specific for a peptide in accordance with claim 6.

10. A pharmaceutical composition in accordance with claim 9 comprising an effective amount of a peptide in accordance with claim 6 and a pharmaceutically acceptable carrier.

11. A pharmaceutical composition in accordance with claim 9 comprising an effective amount of a protein in accordance with any of claims 4 or 5 and a pharmaceutically acceptable carrier.

12. A pharmaceutical composition in accordance with claim 9 comprising an effective amount of an antibody specific for a peptide in accordance with claim 6 and a pharmaceutically acceptable carrier.

13. A method of controlling locomotion of vertebrate cells *in vitro* comprising contacting the cells with an effective amount of one of the following agents:
(i) an antibody specific for a peptide in accordance with claim 6;
(ii) a peptide in accordance with claim 6;
(iii) a protein according to claims 4 or 5 or a fragment thereof.

14. A method of controlling locomotion of cells *in vitro* comprising administering to the cells an effective amount of a protein or a peptide according to any one of claims 4 to 6 or a fragment thereof which retains the ability to bind hyaluronan.

15. A method of inhibiting fertilisation of ova by sperm in a non-human vertebrate comprising contacting the sperm with at least one antibody specific for a protein or peptide according to any one of claims 4 to 6 or a fragment thereof which retains the ability to bind hyaluronan.

16. A method of restoring function *in vitro* in a mammalian cell which is deficient in a protein according to claim 4 or claim 5, comprising transfecting the cell with a DNA sequence in accordance with any of claims 1 to 4.

17. A method of detecting a disease or physiological state associated with altered expression of a protein according to claim 4 or claim 5, in the cells of a vertebrate comprising determining the presence or amount of the protein or a fragment thereof in a sample containing the cells.

18. Use of a reagent selected from:
(i) an antibody specific for a peptide in accordance with claim 6;
(ii) a peptide in accordance with claim 6;
(iii) a protein in accordance with any of claims 4 or 5 or fragment thereof,
in the manufacture of a medicament to treat diseases involving cell locomotion.

19. Use of a peptide in accordance with claim 6 in the manufacture of a medicament to treat diseases involving cell locomotion.

20. Use of a protein or peptide according to claim 4 or a fragment thereof which retains the ability to bind hyaluronan in the manufacture of a medicament to treat diseases involving cell locomotion.

21. Use of a DNA sequence according to any of claims 1 to 3 in the manufacture of a medicament to treat a cell which is deficient in a protein according to claim 4 or claim 5 or a fragment thereof which retains the ability to bind hyaluronan.

## Patentansprüche

1. Isoliertes DNA-Molekül, umfassend eine Nucletidsequenz, die ein Fortbewegungs-assoziiertes Hyaluronan bindendes Protein kodiert, wobei die Nucleotidsequenz eine der nachfolgenden Sequenzen ist:
(i) Die Nucleotidsequenz der Figuren 23A, 23B und 23C (Sequenz ID No. 1);
(ii) Die Nucleotidsequenz 147 - 1428 der Figuren 23A, 23B und 23C.

2. DNA-Molekül gemäß Anspruch 1, kodierend die abgeleitete Aminosäuresequenz der Figuren 23A, 23B und 23C (Sequenz ID No. 2), beginnend mit dem ersten Methionin, oder ein Fragment davon, welches die Fähigkeit aufweist, Hyaluronan zu binden.

3. DNA-Molekül gemäß Anspruch 1, kodierend die abgeleitete Aminosäuresequenz der Figuren 23A, 23B und 23C, beginnend mit dem zweiten Methionin, oder ein Fragment davon, welches die Fähigkeit aufweist, Hyaluronan zu binden.

4. Isoliertes Fortbewegungs-assoziiertes Hyaluronan bindendes Protein, umfassend die abgeleitete Aminosäuresequenz der Figuren 23A, 23B und 23C oder ein Fragment davon, welches die Fähigkeit aufweist, Hyaluronan zu binden.

5. Protein gemäß Anspruch 4, umfassend die Aminosäuresequenz der Figuren 23A, 23B und 23C, beginnend mit dem zweiten Methionin, oder ein Fragment davon, welches die Fähigkeit aufweist, Hyaluronan zu binden.

6. Hyaluronan bindendes Peptid, wobei das Peptid eines der folgenden Peptide ist:
(i) 
Lys - Gln - Lys - Ile - Lys - His - Val - Val -
Lys;
(ii) 
Lys - Ile - Lys - His - Val - Val - Lys - Leu -
Lys;
(iii) 
Lys - Leu - Arg - Ser - Gln - Leu - Val - Lys -
Arg;
(iv)

Lys - Leu - Arg - Ser - Gln - Leu - Val - Lys -
Arg - Lys.

7. Isoliertes DNA-Molekül, das ein Peptid gemäß Anspruch 6 kodiert.

8. Antikörper, der spezifisch für ein Peptid gemäß Anspruch 6 ist.

9. Pharmazeutische Zusammensetzung, umfassend eine wirksame Menge eines der nachfolgenden Stoffe und einen pharmazeutisch zulässigen Träger:
(i) Ein Protein gemäß einem der Ansprüche 4 oder 5,
(ii) Ein Peptid gemäß Anspruch 6,
(iii) Ein Antikörper, der spezifisch für ein Peptid gemäß Anspruch 6 ist.

10. Pharmazeutische Zusammensetzung gemäß Anspruch 9, umfassend eine wirksame Menge eines Peptids gemäß Anspruch 6 und einen pharmazeutisch zulässigen Träger.

11. Pharmazeutische Zusammensetzung gemäß Anspruch 9, umfassend eine wirksame Menge eines Peptids gemäß einem der Ansprüche 4 oder 5 und einen pharmazeutisch zulässigen Träger.

12. Pharmazeutische Zusammensetzung gemäß Anspruch 9, umfassend eine wirksame Menge eines Antikörpers, welcher spezifisch für ein Peptid gemäß Anspruch 6 ist, und einen pharmazeutisch zulässigen Träger.

13. Verfahren zur Steuerung der Fortbewegung von Wirbeltierzellen *in vitro,* umfassend das Kontaktieren der Zellen mit einer wirksamen Menge eines der nachfolgenden Agenzien:
(i) Ein Antikörper, der spezifisch für ein Peptid gemäß Anspruch 6 ist,
(ii) Ein Peptid gemäß Anspruch 6,
(iii) Ein Protein gemäß einem der Ansprüche 4 oder 5 oder ein Fragment davon.

14. Verfahren zur Steuerung der Fortbewegung von Zellen *in vitro,* umfassend den Zellen eine wirksame Menge eines Proteins oder Peptids gemäß einem der Ansprüche 4 bis 6 oder eines Fragments davon, welches die Fähigkeit aufweist, Hyaluronan zu binden, zu verabreichen.

15. Verfahren zur Hemmung der Befruchtung von Eizellen durch Sperma in einem nicht-menschlichen Wirbeltier, umfassend die Kontaktierung des Spermas mit zumindest einem Antikörper, der spezifisch für ein Protein oder Peptid gemäß einem der Ansprüche 4 bis 6 ist, oder für ein Fragment davon, welches die Fähigkeit aufweist, Hyaluronan zu binden.

16. Verfahren zur Wiederherstellung von Funktionen *in vitro* in einer Säugetierzelle, welche mangelhaft in einem Protein gemäß einem der Ansprüche 4 oder 5 ist, umfassend das Transfektieren der Zell mit einer DNA-Sequenz gemäß einem der Ansprüche 1 bis 4.

17. Verfahren zur Erkennung einer Erkrankung oder eines physiologischen Stadiums, welches mit einer veränderten Expression eines Proteins gemäß einem der Ansprüche 4 oder 5 verbunden ist, in den Zellen eines Wirbeltieres, umfassend die Bestimmung der Anwesenheit oder der Menge des Proteins oder eines Fragments davon in einer die Zellen enthaltenden Probe.

18. Verwendung eines Reagenz ausgewählt aus:
(i) Einem Antikörper, der spezifisch für ein Peptid gemäß Anspruch 6 ist,
(ii) einem Peptid gemäß Anspruch 6,
(iii) einem Protein gemäß einem der Ansprüche 4 oder 5 oder einem Fragment davon,
bei der Herstellung eines Medikamentes zur Behandlung von Erkrankungen, in welchen eine Zellfortbewegung involviert ist.

19. Verwendung eines Peptids gemäß Anspruch 6 bei der Herstellung eines Medikaments zur Behandlung von Erkrankungen, in welchen eine Zellfortbewegung involviert ist.

20. Verwendung eines Proteins oder Peptids gemäß Anspruch 4 oder eines Fragments davon, welches die Fähigkeit aufweist, Hyaluronan zu binden, bei der Herstellung eines Medikaments zur Behandlung von Erkrankungen, in welchen eine Zellfortbewegung involviert ist.

21. Verwendung einer DNA-Sequenz gemäß einem der Ansprüche 1 bis 3 bei der Herstellung eines Medikamentes zur Behandlung einer Zelle, die an einem Protein gemäß einem der Ansprüche 4 oder 5 oder einem Fragment davon, welches die Fähigkeit aufweist, Hyaluronan zu binden, mangelhaft ist.

## Revendications

1. Molécule d'ADN isolée comprenant une séquence de nucléotides codant une protéine liant l'hyaluronane associé à la locomotion, dans laquelle la séquence de nucléotides est l'une des séquences suivantes :
(i) la séquence de nucléotides des figures 23A, 23B et 23C (séquence ID n°1) ;
(ii) la séquence de nucléotides 147 à 1428 des figures 23A, 23B et 23C.

2. Molécule d'ADN selon la revendication 1, codant la séquence d'acides aminés dérivée de la figure 23A, 23B et 23C (séquence ID n°2), en commençant par la première méthionine, ou un fragment de celle-ci qui a conservé la capacité à lier l'hyaluronane.

3. Molécule d' ADN selon la revendication 1, codant la séquence d'acides aminés dérivée des figures 23A, 23B et 23C, en commençant par la seconde méthionine, ou un fragment de celle-ci qui a conservé la capacité à lier l'hyaluronane.

4. Protéine isolée liant l'hyaluronane associé à la locomotion, comprenant la séquence d'acides aminés dérivée des figures 23A, 23B et 23C, ou un fragment de celle-ci qui a conservé la capacité à lier l'hyaluronane.

5. Protéine selon la revendication 4, comprenant la séquence d'acides aminés des figures 23A, 23B et 23C, en commençant par la seconde méthionine, ou un fragment de celle-ci qui conserve la capacité à lier l'hyaluronane.

6. Peptide liant l'hyaluronane, dans lequel le peptide est l'un des peptides suivants :
(i) Lys - Gln - Lys - Ile - Lys - His - Val - Val - Lys ;
(ii) 
Lys - Ile - Lys - His - Val - Val - Lys - Leu -
Lys ;
(iii) 
Lys - Leu - Arg - Ser - Gln - Leu - Val - Lys -
Arg ;
(iv)

Lys - Leu - Arg - Ser - Gln - Leu - Val - Lys - Arg
- Lys.

7. Molécule d'ADN isolée codant un peptide selon la revendication 6.

8. Anticorps spécifique d'un peptide selon la revendication 6.

9. Composition pharmaceutique comprenant une quantité efficace de l'un des agents suivants et un support pharmaceutiquement acceptable :
(i) une protéine selon l'une quelconque des revendications 4 ou 5 ;
(ii) un peptide selon la revendication 6 ;
(iii) un anticorps spécifique d'un peptide selon la revendication 6.

10. Composition pharmaceutique selon la revendication 9, comprenant une quantité efficace d'un peptide selon la revendication 6 et un support pharmaceutiquement acceptable.

11. Composition pharmaceutique selon la revendication 9, comprenant une quantité efficace d'une protéine selon l'une quelconque des revendications 4 ou 5 et un support pharmaceutiquement acceptable.

12. Composition pharmaceutique selon la revendication 9, comprenant une quantité efficace d'un anticorps spécifique d'un peptide selon la revendication 6 et un support pharmaceutiquement acceptable.

13. Procédé de contrôle de la locomotion des cellules de vertébrés *in vitro,* comprenant la mise en contact des cellules avec une quantité efficace de l'un des agents suivants :
(i) un anticorps spécifique d'un peptide selon la revendication 6 ;
(ii) un peptide selon la revendication 6 ;
(iii) une protéine selon l'une quelconque des revendications 4 ou 5, ou un fragment de celle-ci.

14. Procédé de contrôle de la locomotion des cellules *in vitro,* comprenant l'administration aux cellules d'une quantité efficace d'une protéine ou d'un peptide selon l'une quelconque des revendications 4 à 6 ou un fragment de celle-ci ou celui-ci qui a conservé la capacité de lier l'hyaluronane.

15. Procédé d'inhibition de la fertilisation des ovaires par du sperme chez un vertébré non humain, comprenant la mise en contact du sperme avec au moins un anticorps spécifique à une protéine ou d'un peptide selon l'une quelconque des revendications 4 à 6 ou d'un fragment de celle-ci ou celui-ci qui a conservé la capacité de lier l'hyaluronane.

16. Procédé de restauration de fonction *in vitro* chez une cellule de mammifère qui est déficiente en une protéine selon la revendication 4 ou la revendication 5, comprenant la transfection de la cellule avec une séquence d'ADN selon l'une quelconque des revendications 1 à 4.

17. Procédé de détection d'une maladie ou d'un état physiologique associé à l'expression modifiée d'une protéine selon la revendication 4 ou la revendication 5 dans les cellules d'un vertébré, comprenant la détermination de la présence ou de la quantité de la protéine ou d'un fragment de celle-ci dans un échantillon contenant les cellules.

18. Utilisation d'un réactif choisi parmi :
(i) un anticorps spécifique d'un peptide selon la revendication 6 ;
(ii) un peptide selon la revendication 6 ;
(iii) une protéine selon l'une quelconque des revendications 4 ou 5, ou un fragment de celle-ci,
pour la fabrication d'un médicament destiné à traiter des maladies impliquant la locomotion cellulaire.

19. Utilisation d'un peptide selon la revendication 6 pour la fabrication d'un médicament destiné à traiter des maladies impliquant la locomotion cellulaire.

20. Utilisation d'une protéine ou d'un peptide selon la revendication 4, ou d'un fragment de celle-ci ou celui-ci qui a conservé la capacité à lier l'hyaluronane, pour la fabrication d'un médicament destiné à traiter des maladies impliquant la locomotion cellulaire.

21. Utilisation d'une séquence d'ADN selon l'une quelconque des revendications 1 à 3, pour la fabrication d'un médicament destiné à traiter une cellule qui est déficiente en une protéine selon la revendication 4 ou la revendication 5 ou en un fragment de celle-ci qui a conservé la capacité à lier l'hyaluronane.
